# EUROPÄISCHE PATENTSCHRIFT

(11) **EP 1 473 293 B1**
(45) Veröffentlichungstag und Bekanntmachung des Hinweises auf die Patenterteilung: **19.03.2008**
(21) Anmeldenummer: 04007549.1
(22) Anmeldetag: 29.03.2004
(51) Int. Cl.: C07D 405/06, C07D 417/06, C07D 407/06, A61K 31/4178, A61P 35/00

(54) **Chromenonderivate**
Chromenon derivatives
Dérivés de chroménones.

(30) Priorität: 30.04.2003 DE 10319552; 05.08.2003 DE 10335782
(43) Veröffentlichungstag der Anmeldung: 03.11.2004
(73) Patentinhaber: Merck Patent GmbH, 64293 Darmstadt (DE)
(72) Erfinder: Mujica-Fernaud, Teresa, Dr., 64291 Darmstadt (DE); Buchholz, Herwig, Dr., 60599 Frankfurt (DE); Rautenberg, Wilfried, Dr., 64354 Reinheim (DE); Sirrenberg, Christian, Dr., 64289 Darmstadt (DE)

(56) Entgegenhaltungen:
- WO-A-20/04022561
- FR-A- 2 189 063
- S. YOUNES-EL-HAGE ET AL.: "synthèse et étude de l'activité antidépressive d'hétéroaryl carboxamides de la benzylpipérazine" ANNALES PHARMACEUTIQUES FRANCAISES., Bd. 58, Nr. 4, 2000, Seiten 254-9, XP008031993 FRMASSON ET CIE. PARIS.
- HADJERI MOHAMED ET AL: "Modulation of P-glycoprotein-mediated multidrug resistance by flavonoid derivatives and analogues." JOURNAL OF MEDICINAL CHEMISTRY, [Online] Bd. 46, Nr. 11, 19. April 2003 (2003-04-19), Seiten 2125-2131, XP002285928 ISSN: 0022-2623 Journal Artikel Gefunden im Internet: URL:http://dx.doi.org/10.1021/jm021099i> [gefunden am 2004-06-24]
- S.HOOSEINIMEHR ET AL.: "radioprotective effects of 2-imino-3-[(chromone-2-yl)carbonyl]thiazol idine" JOURNAL OF RADIATION RESEARCH, Bd. 43, Nr. 3, 2002, Seiten 293-300, XP008031990

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der Tyrosinkinasen und Raf-Kinasen, eine Rolle spielt, ferner pharmazeutische Zusammensetzungen, die diese Verbindungen enthalten, sowie die Verwendung der Verbindungen zur Behandlung von kinasebedingter Krankheiten.

Im einzelnen betrifft die vorliegende Erfindung Verbindungen, die die Signaltransduktion der Tyrosinkinasen hemmen, regulieren und/oder modulieren, Zusammensetzungen, die diese Verbindungen enthalten, sowie Verfahren zu ihrer Verwendung zur Behandlung von tyrosinkinasebedingten Krankheiten und Leiden wie Krebs, Tumorwachstum, Arteriosklerose, altersbedingte Makula-Degeneration, diabetische Retinopathie, Entzündungserkrankungen und dergleichen bei Säugetieren. Bei den Tyrosinkinasen handelt es sich um eine Klasse von Enyzmen, die die Übertragung des endständigen Phosphats des Adenosintriphosphats auf Tyrosinreste bei Proteinsubstraten katalysieren. Man nimmt an, dass den Tyrosinkinasen bei verschiedenen Zellfunktionen über die Substratphosphorylierung eine wesentliche Rolle bei der Signaltransduktion zukommt. Obwohl die genaue Mechanismen der Signaltransduktion noch unklar sind, wurde gezeigt, dass die Tyrosinkinasen wichtige Faktoren bei der Zellproliferation, der Karzinogenese und der Zelldifferenzierung darstellen.

Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen.

Die Rezeptor-Tyrosinkinasen bestehen aus einer Vielzahl von Transmembranrezeptoren mit unterschiedlicher biologischer Wirksamkeit. So wurden ungefähr 20 verschiedene Unterfamilien von Rezeptor-Tyrosinkinasen identifiziert. Eine Tyrosinkinase-Unterfamilie, die die Bezeichnung HER-Unterfamilie trägt, besteht aus EGFR, HER2, HER3 und HER4. Zu den Liganden dieser Rezeptor-Unterfamilie zählen der Epithel-Wachstumsfaktor, TGF-α, Amphiregulin, HB-EGF, Betacellulin und Heregulin. Die Insulin-Unterfamilie, zu der INS-R, IGF-IR und IR-R zählen, stellt eine weitere Unterfamilie dieser Rezeptor-Tyrosinkinasen dar. Die PDGF-Unterfamilie beinhaltet den PDGF-α- and -β-Rezeptor, CSFIR, c-kit und FLK-II. Außerdem gibt es die FLK-Familie, die aus dem Kinaseinsertdomänenrezeptor (KDR), der fötalen Leberkinase-1 (FLK-1), der fötalen Leberkinase-4 (FLK-4) und der fms-Tyrosinkinase-1 (flt-1) besteht. Die PDGF- und FLK-Familie werden üblicherweise aufgrund der zwischen den beiden Gruppen bestehenden Ähnlichkeiten gemeinsam diskutiert. Für eine genaue Diskussion der Rezeptor-Tyrosinkinasen siehe die Arbeit von Plowman et al., DN & P 7(6):334-339, 1994, die hiermit durch Bezugnahme aufgenommen wird.

Die zytosolischen Tyrosinkinasen bestehen ebenfalls aus einer Vielzahl von Unterfamilien, darunter Src, Frk, Btk, Csk, Abl, Zap70, Fes/Fps, Fak, Jak, Ack, and LIMK. Jede dieser Unterfamilien ist weiter in verschiedene Rezeptoren unterteilt. So stellt zum Beispiel die Src-Unterfamilie eine der größten Unterfamilien dar. Sie beinhaltet Src, Yes, Fyn, Lyn, Lck, Blk, Hck, Fgr und Yrk. Die Src-Enzymunterfamilie wurde mit der Onkogenese in Verbindung gebracht. Für eine genauere Diskussion der zytosolischen Tyrosinkinasen, siehe die Arbeit von Bolen Oncogene, 8:2025-2031 (1993), die hiermit durch Bezugnahme aufgenommen wird.

Sowohl die Rezeptor-Tyrosinkinasen als auch die zytosolischen Tyrosinkinasen sind an Signalübertragungswegen der Zelle, die zu verschiedenen Leidenszuständen führen, darunter Krebs, Schuppenflechte und Hyperimmunreaktionen, beteiligt.

Es wurde vorgeschlagen, dass verschiedene Rezeptor-Tyrosinkinasen sowie die an sie bindenden Wachstumsfaktoren eine Rolle bei den Angiogenese spielen, obwohl einige die Angiogenese indirekt fördern könnten (Mustonen und Alitalo, J. Cell Biol. 129:895-898, 1995). Eine dieser Rezeptor-Tyrosinkinasen ist die fötale Leberkinase 1, auch FLK-1 genannt. Das menschliche Analog der FLK-1 ist der kinase-insertdomänenhaltige Rezeptor KDR, der auch unter der Bezeichnung Gefäßendothelzellenwachstumsfaktorrezeptor 2 bzw. VEGFR-2 bekannt ist, da er VEGF hochaffin bindet. Schließlich wurde die Maus-Version dieses Rezeptors auch ebenfalls NYK genannt (Oelrichs et al., Oncogene 8(1):11-15, 1993). VEGF und KDR stellen ein Ligand-Rezeptor-Paar dar, das eine wesentliche Rolle bei der Proliferation der Gefäßendothelzellen und der Bildung und Sprossung der Blutgefäße, die als Vaskulogenese bzw. Angiogenese bezeichnet werden, spielt.

Die Angiogenese ist durch eine übermäßig starke Aktivität des Gefäßendothelwachstumsfaktors (VEGF) gekennzeichnet. Der VEGF besteht eigentlich aus einer Familie von Liganden (Klagsburn und D'Amore, Cytokine & Growth Factor Reviews 7:259-270, 1996). Der VEGF bindet den hochaffinen transmembranösen Tyrosinkinaserezepzor KDR und die verwandte fms-Tyrosinkinase-1, auch unter der Bezeichnung Flt-1 oder Gefäßendothelzellenwachstumsfaktorrezeptor 1 (VEGFR-1) bekannt. Aus Zellkultur- und Gen- Knockout-Versuchen geht hervor, dass jeder Rezeptor zu unterschiedlichen Aspekten der Angiogenese beiträgt. Der KDR führt die mitogene Funktion des VEGF herbei, während Flt-1 nichtmitogene Funktionen, wie diejenigen, die mit der Zelladhäsion in Zusammenhang stehen, zu modulieren scheint. Eine Hemmung des KDR moduliert daher das Niveau der mitogenen VEGF-Aktivität. Tatsächlich wurde gezeigt, dass das Tumorwachstum von der antiangiogenen Wirkung der VEGF-Rezeptor-Antagonisten beeinflusst wird (Kim et al., Nature 362, S. 841- 844, 1993).

Feste Tumore können daher mit Tyrosinhemmern behandelt werden, da diese Tumore für die Bildung der zur Unterstützung ihres Wachstums erforderlichen Blutgefäße auf Angiogenese angewiesen sind. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom. Zu weiteren Beispielen zählen Karzinome, bei denen eine Überexpression oder Aktivierung von Rafaktivierenden Onkogenen (z.B. K-ras, erb-B) beobachtet wird. Zu diesen Karzinomen zählen Bauchspeicheldrüsen- und Brustkarzinom.

Hemmstoffe dieser Tyrosinkinasen eignen sich daher zur Vorbeugung und Behandlung von proliferativen Krankheiten, die durch diese Enzyme bedingt sind.

Die angiogene Aktivität des VEGF ist nicht auf Tumore beschränkt. Der VEGF ist für die bei diabetischer Retinopathie in bzw. in der Nähe der Retina produzierte angiogene Aktivität verantwortlich. Dieses Gefäßwachstum in der Retina führt zu geschwächter Sehkraft und schließlich Erblindung. Die VEGF-mRNA- und -protein-Spiegel im Auge werden durch Leiden wie Netzhautvenenokklusion beim Primaten sowie verringertem pO₂-Spiegel bei der Maus, die zu Gefäßneubildung führen, erhöht. Intraokular injizierte monoklonale Anti-VEGF-Antikörper, oder VEGF-Rezeptor-Immunkonjugate, hemmen sowohl im Primaten- als auch im Nagetiermodell die Gefäßneubildung im Auge. Unabhängig vom Grund der Induktion des VEGF bei der diabetischen Retinopathie des Menschen, eignet sich die Hemmung des Augen-VEGF zur Behandlung dieser Krankheit.

Die VEGF-Expression ist auch in hypoxischen Regionen von tierischen und menschlichen Tumoren neben Nekrosezonen stark erhöht. Der VEGF wird außerdem durch die Expression der Onkogene ras, raf, src und p53-Mutante (die alle bei der Bekämpfung von Krebs von Bedeutung sind) hinaufreguliert. Monoklonale Anti-VEGF-Antikörper hemmen bei der Nacktmaus das Wachstum menschlicher Tumore. Obwohl die gleichen Tumorzellen in Kultur weiterhin VEGF exprimieren, verringern die Antikörper ihre Zellteilungsrate nicht. So wirkt der aus Tumoren stammende VEGF nicht als autokriner mitogener Faktor. Der VEGF trägt daher in vivo dadurch zum Tumorwachstum bei, dass er durch seine parakrine Gefäßendothelzellen-Chemotaxis- und -Mitogeneseaktivität die Angiogenese fördert. Diese monoklonalen Antikörper hemmen auch das Wachstum von typischerweise weniger stark vaskularisierten Human-Kolonkarzinomen bei thymuslosen Mäusen und verringern die Anzahl der aus inokulierten Zellen entstehenden Tumore.

Die Expression eines VEGF-bindenden Konstrukts von Flk-1, Flt-1, dem zur Entfernung der zytoplasmatischen Tyrosinkinasedomänen, jedoch unter Beibehaltung eines Membranankers, verkürzten Maus-KDR-Rezeptorhomologs, in Viren stoppt praktisch das Wachstum eines transplantierbaren Glioblastoms bei der Maus, vermutlich aufgrund des dominant-negativen Mechanismus der Heterodimerbildung mit transmembranösen Endothelzellen-VEGF-Rezeptoren. Embryostammzellen, die in der Nacktmaus üblicherweise in Form von festen Tumoren wachsen, bilden bei Knock-out aller beider VEGF-Allele keine nachweisbaren Tumore. Aus diesen Daten gemeinsam geht die Rolle des VEGF beim Wachstum fester Tumore hervor. Die Hemmung von von KDR bzw. Flt-1 ist an der pathologischen Angiogenese beteiligt, und diese Rezeptoren eignen sich zur Behandlung von Krankheiten, bei denen Angiogenese einen Teil der Gesamtpathologie, z.B. Entzündung, diabetische Retina-Vaskularisierung sowie verschiedene Formen von Krebs, darstellt, da bekannt ist, dass das Tumorwachstum angiogeneseabhängig ist (Weidner et al., N. Engl. J. Med., 324, S. 1-8, 1991).

Die vorliegende Erfindung betrifft weiterhin die Verbindungen als Inhibitoren von Raf-Kinasen.

Protein-Phosphorylierung ist ein fundamentaler Prozess für die Regulation von Zellfunktionen. Die koordinierte Wirkung von sowohl Proteinkinasen als auch Phosphatasen kontrolliert die Phosphorylierungsgrade und folglich die Aktivität spezifischer Zielproteine. Eine der vorherrschenden Rollen der Protein-Phosphorylierung ist bei der Signaltransduktion, wenn extrazelluläre Signale amplifiziert und durch eine Kaskade von Protein-Phosphorylierungs- und Dephosphorylierungsereignissen, z. B. im p21^{ras}/raf-Weg propagiert werden.

Das p21^{ras}-Gen wurde als ein Onkogen der Harvey- und Kirsten-Ratten-Sarkom-Viren (H-Ras bzw. K-Ras) entdeckt. Beim Menschen wurden charakteristische Mutationen im zellulären Ras-Gen (c-Ras) mit vielen verschiedenen Krebstypen in Verbindung gebracht. Von diesen mutanten Allelen, die Ras konstitutiv aktiv machen, wurde gezeigt, dass sie Zellen, wie zum Beispiel die murine Zelllinie NIH 3T3, in Kultur transformieren.

Das p21^{ras}-Onkogen ist ein wichtiger beitragender Faktor bei der Entwicklung und Progression humaner solider Karzinome und ist bei 30 % aller humaner Karzinome mutiert (Bolton et al. (1994) Ann. Rep. Med. Chem., 29, 165-74; Bos. (1989) Cancer Res., 49, 4682-9). In seiner normalen, nicht mutierten Form ist das Ras-Protein ein Schlüsselelement der Signaltransduktionskaskade, die durch Wachstumsfaktor-Rezeptoren in fast allen Geweben gesteuert wird (Avruch et al. (1994) Trends Biochem. Sci., 19, 279-83).

Biochemisch ist Ras ein Guanin-Nukleotid-bindendes Protein, und das Zyklieren zwischen einer GTP-gebundenen aktivierten und einer GDPgebundenen ruhenden Form wird von Ras-endogener GTPase-Aktivität und anderen Regulatorproteinen strikt kontrolliert. Das Ras-Genprodukt bindet an Guanintriphosphat (GTP) und Guanindiphosphat (GDP) und hydrolysiert GTP zu GDP. Ras ist im GTP-gebundenen Zustand aktiv. In den Ras-Mutanten in Krebszellen ist die endogene GTPase-Aktivität abgeschwächt, und folglich gibt das Protein konstitutive Wachstumssignale an "Downstream"-Effektoren, wie zum Beispiel an das Enzym Raf-Kinase ab.

Dies führt zum krebsartigen Wachstum der Zellen, die diese Mutanten tragen (Magnuson et al. (1994) Semin. Cancer Biol., 5, 247-53). Das Ras-Proto-Onkogen benötigt ein funktionell intaktes C-Raf-1-Proto-Onkogen, um in höheren Eukaryoten durch Rezeptor- und Nicht-Rezeptor-Tyrosin-Kinasen initiierte Wachstums- und Differenzierungssignale zu transduzieren.

Aktiviertes Ras ist für die Aktivierung des C-Raf-1-Proto-Onkogens notwendig, die biochemischen Schritte, durch die Ras die Raf-1-Protein-(Ser/Thr)-Kinase aktiviert, sind jedoch inzwischen gut charakterisiert. Es wurde gezeigt, dass das Inhibieren des Effekts von aktivem Ras durch Inhibition des Raf-Kinase-Signalwegs mittels Verabreichung von deaktivierenden Antikörpern gegen Raf-Kinase oder mittels Koexpression dominanter negativer Raf-Kinase oder dominanter negativer MEK (MAPKK), dem Substrat der Raf-Kinase, zur Reversion transformierter Zellen zum normalen Wachstumsphänotyp führt, siehe: Daum et al. (1994) Trends Biochem. Sci., 19, 474-80; Fridman et al. (1994) J Biol. Chem., 269, 30105-8. Kolch et al. (1991) Nature, 349, 426-28) und zur Besprechung Weinstein-Oppenheimer et al. Pharm. & Therap. (2000), 88, 229-279.

Auf ähnliche Weise wurde die Inhibition von Raf-Kinase (durch Antisense-Oligodesoxynukleotide) in vitro und in vivo mit der Inhibition des Wachstums einer Reihe verschiedener humaner Tumortypen in Beziehung gebracht (Monia et al., Nat. Med. 1996, 2, 668-75).

Raf-Serin- und Threonin-spezifische Protein-Kinasen sind cytosolische Enzyme, die das Zellwachstum in einer Reihe verschiedener Zellsysteme stimulieren (Rapp, U.R., et al. (1988) in The Oncogene Handbook; T. Curran, E.P. Reddy und A. Skalka (Hrsg.) Elsevier Science Publishers; Niederlande, S. 213-253; Rapp, U.R., et al. (1988) Cold Spring Harbor Sym. Quant. Biol. 53:173-184; Rapp, U.R., et al. (1990) Inv Curr. Top. Microbiol. Immunol. Potter und Melchers (Hrsg.), Berlin, Springer-Verlag 166:129-139).

Drei Isozyme wurden charakterisiert:

C-Raf (Raf-1) (Bonner, T.I., et al. (1986) Nucleic Acids Res. 14:1009-1015). A-Raf (Beck, T.W., et al. (1987) Nucleic Acids Res. 15:595-609), und B-Raf (Qkawa, S., et al. (1998) Mol. Cell. Biol. 8:2651-2654; Sithanandam, G. et al. (1990) Oncogene:1775). Diese Enzyme untercheiden sich durch ihre Expression in verschiedenen Geweben. Raf-1 wird in allen Organen und in allen Zelllinien, die untersucht wurden, exprimiert, und A- und B-Raf werden in Urogenital- bzw. Hirngeweben exprimiert (Storm, S.M. (1990) Oncogene 5:345-351).

Raf-Gene sind Proto-Onkogene: Sie können die maligne Transformation von Zellen initiieren, wenn sie in spezifisch veränderten Formen exprimiert werden. Genetische Veränderungen, die zu onkogener Aktivierung führen, erzeugen eine konstitutiv aktive Proteinkinase durch Entfernung oder Interferenz mit einer N-terminalen negativen Regulatordomäne des Proteins (Heidecker, G., et al. (1990) Mol. Cell. Biol. 10:2503-2512; Rapp, U.R., et al. (1987) in Oncogenes and Cancer; S. A. Aaronson, J. Bishop, T. Sugimura, M. Terada, K. Toyoshima und P. K. Vogt (Hrsg.) Japan Scientific Press, Tokyo). Mikroinjektion in NIH 3T3-Zellen von onkogen aktivierten, aber nicht Wildtyp-Versionen des mit Expressionsvektoren von Escherichia coli präparierten Raf-Proteins führt zu morphologischer Transormation und stimuliert die DNA-Synthese (Rapp, U.R., et al. (1987) in Oncogenes and Cancer; S. A. Aaronson, J. Bishop, T. Sugimura, M. Terada, K. Toyoshima, und P. K. Vogt (Hrsg.) Japan Scientific Press, Tokyo; Smith, M. R., et al. (1990) Mol. Cell. Biol. 10:3828-3833).

Folglich ist aktiviertes Raf-1 ein intrazellulärer Aktivator des Zellwachstums. Raf-1-Protein-Serin-Kinase ist ein Kandidat für den "Downstream"-Effektor der Mitogen-Signaltransduktion, da Raf-Onkogene dem Wachstumsarrest begegnen, der aus einer Blockade zellulärer Ras-Aktivität aufgrund einer zellulären Mutation (Ras-revertante Zellen) oder Mikroinjektion von Anti-Ras-Antikörpern resultiert (Rapp, U.R., et al. (1988) in The Oncogene Handbook, T. Curran, E.P. Reddy und A. Skalka (Hrsg.), Elsevier Science Publishers; Niederlande, S. 213-253; Smith, M.R., et al. (1986) Nature (London) 320:540-543).

Die C-Raf-Funktion ist für die Transformation durch eine Reihe verschiedener Membran-gebundener Onkogene und für die Wachstumsstimulation durch in Sera enthaltene Mitogene erforderlich (Smith, M.R., et al. (1986) Nature (London) 320:540-543). Raf-1-Protein-Serin-Kinase-Aktivität wird durch Mitogene über die Phosphorylierung reguliert (Morrison, D.K., et al. (1989) Cell 58:648-657), welche auch die subzelluläre Verteilung bewirkt (Olah, Z., et al. (1991) Exp. Brain Res. 84:403; Rapp, U.R., et al. (1988) Cold Spring Harbor Sym. Quant. Biol. 53:173-184. Zu Raf-1-aktivierenden Wachstumsfaktoren zählen der aus Thrombozyten stammende Wachstumsfaktor (PDGF) (Morrison, D.K., et al. (1988) Proc. Natl. Acad. Sci. USA 85:8855-8859), der Kolonien-stimulierende Faktor (Baccarini, M., et al. (1990) EMBO J. 9:3649-3657), Insulin (Blackshear, P.J., et al. (1990) J. Biol. Chem. 265:12115-12118), der epidermale Wachstumsfaktor (EGF) (Morrison, R.K., et al. (1988) Proc. Natl. Acad. Sci. USA 85:8855-8859), Interleukin-2 (Turner, B.C., et al. (1991) Proc. Natl. Acad. Sci. USA 88:1227) und Interleukin-3 und der Granulozyten-Makrophagen-Kolonien-stimulierende Faktor (Carroll, M.P., et al. (1990) J. Biol. Chem. 265:19812-19817).

Nach der Mitogen-Behandlung von Zellen transloziert die transient aktivierte Raf-1-Protein-Serin-Kinase in den perinukleären Bereich und den Nukleus (Olah, Z., et al. (1991) Exp. Brain Res. 84:403; Rapp, U.R., et al. (1988) Cold Spring Habor Sym. Quant. Biol. 53:173-184). Zellen, die aktiviertes Raf enthalten, sind in ihrem Genexpressionsmuster verändert (Heidecker, G., et al. (1989) in Genes and signal transduction in multistage carcinogenesis, N. Colburn (Hrsg.), Marcel Dekker, Inc., New York, S. 339-374) und Raf-oncogenes activate transcription from Ap-I/PEA3-dependent promotors in transient transfection assays (Jamal, S., et al. (1990) Science 344:463-466; Kaibuchi, K., et al. (1989) J. Biol. Chem. 264:20855-20858; Wasylyk, C., et al. (1989) Mol. Cell. Biol. 9:2247-2250).

Es gibt mindestens zwei unabhängige Wege für die Raf-1-Aktivierung durch extrazelluläre Mitogene: Einen, der Proteinkinase C (KC) beinhaltet, und einen zweiten, der durch Protein-Tyrosin-Kinasen initiiert wird (Blackshear, P.J., et al. (1990) J. Biol. Chem. 265:12131-12134; Kovacina, K.S., et al. (1990) J. Biol. Chem. 265:12115-12118; Morrison, D.K., et al. (1988) Proc. Natl. Acad. Sci. USA 85:8855-8859; Siegel, J.N., et al. (1990) J. Biol. Chem. 265:18472-18480; Turner, B.C., et al. (1991) Proc. Natl. Acad. Sci. USA 88:1227). In jedem Fall beinhaltet die Aktivierung Raf-1-Protein-Phosphorylierung. Raf-1-Phosphorylierung kann eine Folge einer Kinase-Kaskade sein, die durch Autophosphorylierung amplifiziert wird, oder kann vollkommen durch Autophosphorylierung hervorgerufen werden, die durch Bindung eines vermutlichen Aktivierungsliganden an die Raf-1-Regulatordomäne, analog zur PKC-Aktivierung durch Diacylglycerol initiiert wird (Nishizuka, Y. (1986) Science 233:305-312).

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Proteinkinase PKB (auch als AKT und RAC-PK bekannt) ist ein Mitglied der AKT/PKB-Familie der Serin-/Threonin-Kinasen, und es wurde gezeigt, dass sie an einer diversen Reihe von Signalwegen bei der humanen Malignität beteiligt ist (Nicholson et al., Cell. Signal., 2002, 14, 381-395). PKB, wie auch andere Mitglieder der AKT/PKB-Familie, ist im Cytosol nicht stimulierter Zellen lokalisiert und transloziert nach Stimulation an die Zellmembran. PKB-Translokation kann durch mehrere Liganden, einschließlich des aus Thrombozyten stammenden Wachstumsfaktors, epidermalen Wachstumfaktors, basischen Fibroblasten-Wachstumsfaktors, Zellstress, wie zum Beispiel Hitzeschock und Hyperosmolarität und auch Insulin, aktiviert werden (Bos, Trends Biochem. Sci., 1995, 20, 441-442), und andere Studien haben gezeigt, dass diese Aktivierung über P13-Kinase abläuft, die Wortmannin-empfindlich ist (Franke et al., Science, 1997, 275, 665-668). Sobald PKB an der Plasmamembran lokalisiert ist, wurde gezeigt, dass sie mehrere Funktionen in der Zelle vermittelt, einschließlich Apoptose, der metabolischen Effekte von Insulin, Induktion von Differenzierung und/oder Proliferation, Proteinsynthese und Stressantworten (Alessi und Cohen, Curr. Opin. Genet. Dev., 1998, 8, 55-62; Downward, Curr. Opin. Cell Biol., 1998, 10, 262-267).

PKB wurde 1991 von drei Gruppen unabhängig geklont (Bellacosa et al., Science, 1991, 254, 274-277; Coffer und Woodgett, Eur. J. Biochem., 1991, 201, 475-481; Jones et al., Cell Regul., 1991, 2, 1001- 1009), ihr Zusammenhang mit dem primären humanen Magenkarzinom wurde jedoch bereits 1987 erkannt (Staal et al., Proc. Natl. Acad. Sci. U S A, 1987, 84, 5034-5037). Sequenzierung von PKBα ließ in den Kinasedomänen Homologie zu den PKA- (ca. 68 %) und PKC-Isozymen (ca. 73 %) erkennen (Jones et al., Proc. Natl. Acad. Sci. U.S.A., 1991, 88, 4171-5), eine Tatsache, die zu ihrer Umbenennung in PKB führte. Es gibt drei zelluläre PKB-Isoformen und zwei Splice-Varianten (PKBα, β, γ, β₁, γ₁; Brazil et al. Trends in Bio Sci, 2001, 26, 657-663). Es wurde gefunden, dass PKBα bei Magenadenokarzinomen und einer Brustkrebs-Zelllinie amplifiziert oder überexprimiert wird (Staal et al., Proc. Natl. Acad. Sci. U.S.A., 1987, 84, 5034-7; Jones et al., Cell Regul., 1991, 2, 1001-9). PKBβ wird bei 3 % des Brust- (Bellacosa et al., Int. J. Cancer, 1995 64, 280-5), 12 % des Pankreas- (Cheng et al., Proc. Natl. Acad. Sci. U.S.A., 1996, 93, 3636-41) und 15 % des Eierstockkrebses amplifiziert oder überexprimiert (Bellacosa et al., Int. J. Cancer, 1995, 64, 280-5; Cheng et al., Proc. Natl. Acad. Sci. U.S.A., 1992, 89, 9267-71).

PKBγ wird bei Estrogenrezeptor-defizientem Brustkrebs und bei Androgenunabhängigen Prostata-Zelllinien überexprimiert (Nakatani et al., J. Biol. Chem. 1999, 274, 21528-32).

Es wurde vorgeschlagen, dass PKB ein an der chromosomalen Umordnung an der Chromosomenbande 14q32 beteiligtes Gen ist. Von diesem Locus ist bekannt, dass er bei humanen T-Zellmalignitäten, wie zum Beispiel bei prolymphozytischen Leukämien und Leukämien gemischter Abstammung im Kindersalter der Umordnung unterliegt (Staal et al., Genomics, 1988, 2, 96-98).

PKB spielt auch eine Rolle bei der Verhinderung des "programmierten Zelltodes" oder der Apoptose durch inhibitorische Phosphorylierung von ASK-1, Bad, Caspase9 und FKHR (Übersicht siehe Nicholson et al., Cell Signaling 2001, 14, 281-395). Es wurde nachgewiesen, dass PKB ein Überlebenssignal (Übersicht siehe Lawlor et al., J. of Cell Science 2001, 114, 2903-2910) für Zellen liefert, um sie vor einer Anzahl von Agenzien, einschließlich UV-Strahlung (Dudek et al., Science, 1997, 275, 661-665), Entzug von IGF1 aus neuronalen Zellen, Ablösung von der extrazellulären Matrix, Stress und Hitzeschock zu schützen (Alessi und Cohen, Curr. Opin. Genet. Dev., 1998, 8, 55-62).

Die dual-spezifische Phosphatase PTEN (Phosphatase and Tensin homologue deleted on Chromosome Ten [Phosphatase und Tensin homolog deletiert an Chromosom zehn]) erhöht den Ptdlns(3, 4, 5)P₃-Spiegel in der Zelle durch Dephosphorylierung von Ptdlns(3, 4, 5)P₃. Ptdlns(3, 4, 5)P₃ bindet an die PH-Domäne (Pleckstrin-Homologie- Domäne) von PKB. Diese Bindung stellt einen wesentlichen Schritt für die Membran-Translokation und Aktivierung von PKB dar. PTEN ist ein in einer großen Fraktion von Glioblastom- und Melanom-Zelllinien, fortgeschrittenen Prostatakarzinomen und endometrialen Karzinomen mutiertes Tumor-Suppressor-Gen. Es ist darüber hinaus bei >80 % der Patienten mit erblichen Leiden, wie zum Beispiel Cowden-Syndrom, Lhermitte-Duclos-Syndrom und Bannayan-Zonana-Syndrom deletiert. Die Patienten weisen mehrere ähnliche Merkmale auf, einschließlich multipler gutartiger Tumoren (Harmatomen) und einer erhöhten Anfälligkeit für Brust- und Schilddrüsenmalignitäten (Di Cristofano et al. Cell, 2000, 100, 387-390).

Von heterozygoten PTEN^{+/-}-Mäusen (heterozygote PTEN^{-/-}-Mäuse sind nicht lebensfähig) hergeleitete Zelllinien weisen erhöhte Ptdlns(3, 4, 5)P₃-Spiegel auf, die mit erhöhter PKB-Aktivität, mit einer gleichzeitig verminderten Sensitivität gegen Apoptose einhergehen (Di Christofano et al. Nat. Genet. 1998, 19, 348-355; Stambolic et al., Cell, 1998, 95, 29-39, Myers et al., Proc. Natl. Acad. Si. U.S.A., 1998, 96 13513-13518).

PKB ist auch zur Promotion der Zellzyklus-Progression durch Inhibition des p21-Zellzyklus-Inhibitors fähig (Zhou et al.; Nat. Cell Biol., 2002,3, 245-252).

Diese Befunde könnten die Überexperession von PKB erklären, die in Krebszellen beobachtet wird, die präferenzielles Überleben und Proliferation der Karzinome durch Verhindern der normalen Progression zur Apoptose ermöglicht.

Zur Zeit gibt es keine bekannten Therapeutika, welche die PKB-Aktivität wirksam inhibieren. Folglich besteht noch immer ein seit langem wahrgenommener Bedarf an zusätzlichen Mitteln, die als Chemotherapeutika zur wirksamen Inhibition der PKB-Funktion zur Aktivierung von proapoptotischen Proteinen bei allen Krebsarten fähig sind.

Die Identifikation von kleinen Verbindungen, die die Signaltransduktion der Tyrosinkinasen und/oder Raf-Kinasen spezifisch hemmen, regulieren und/oder modulieren, ist daher wünschenswert und ein Ziel der vorliegenden Erfindung.

Es wurde gefunden, daß die Verbindungen der Formel I und ihre Salze bei guter Verträglichkeit sehr wertvolle pharmakologische Eigenschaften besitzen.

Insbesondere zeigen sie inhibierende Eigenschaften der Tyrosinkinase. Es wurde weiterhin gefunden, daß die Verbindungen der Formel I, die im Allgemeinen als Chromenon-Derivate beschrieben werden, Inhibitoren des Enzyms Raf-Kinase sind. Da das Enzym ein "Downstream"- Effektor von p21^{ras} ist, erweisen sich die Inhibitoren in pharmazeutischen Zusammensetzungen für die human- oder veterinärmedizinische Anwendung als nützlich, wenn Inhibition des Raf-Kinase-Weges, z. B. bei der Behandlung von Tumoren und/oder durch Raf-Kinase vermitteltem krebsartigen Zellwachstum, angezeigt ist. Die Verbindungen sind insbesondere nützlich bei der Behandlung solider Karzinome bei Mensch und Tier, z. B. von murinem Krebs, da die Progression dieser Krebse abhängig ist von der Ras-Protein-Signaltransduktionskaskade und deshalb auf die Behandlung durch Unterbrechung der Kaskade, d. h. durch Inhibition der Raf-Kinase, anspricht. Dementsprechend wird die Verbindung der Formel I oder ein pharmazeutisch unbedenkliches Salz davon für die Behandlung von Krankheiten verabreicht, die durch den Raf-Kinase-Weg vermittelt werden, besonders Krebs, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom), pathologische Angiogenese und metastatische Zellmigration. Die Verbindungen sind ferner nützlich bei der Behandlung der Komplemenfaktivierungsabhängigen chronischen Entzündung (Niculescu et al. (2002) Immunol. Res., 24:191-199) und durch HIV-1 (Human Immunodeficiency Virus Typ 1) induzierte Immunschwäche (Popik et al. (1998) J Virol, 72: 6406-6413).

Es wurde überraschend gefunden, dass erfindungsgemäße Verbindungen Derivate mit Signalwegen, besonders mit den hierin beschriebenen Signalwegen und bevorzugt dem Raf-Kinase-Signalweg interagieren können. Erfindungsgemäße Chromenon-Derivate zeigen bevorzugt eine vorteilhafte biologische Aktivität, die in auf Enzymen basierenden Assays, zum Beispiel Assays wie hierin beschrieben, leicht nachweisbar ist. In derartigen auf Enzymen basierenden Assays zeigen und bewirken erfindungsgemäße Chromenon-Derivate bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Wie hierin besprochen, sind diese Signalwege für verschiedene Erkrankungen relevant. Dementsprechend sind Chromenon-Derivate nützlich bei der Prophylaxe und/oder Behandlung von Erkrankungen, die von den genannten Signalwegen durch Interaktion mit einem oder mehreren der genannten Signalwege abhängig sind.

Gegenstand der vorliegenden Erfindung sind deshalb erfindungsgemäße Verbindungen als Promotoren oder Inhibitoren, bevorzugt als Inhibitoren der hierin beschriebenen Signalwege. Bevorzugter Gegenstand der Erfindung sind deshalb erfindungsgemäße Verbindungen als Promotoren oder Inhibitoren, bevorzugt als Inhibitoren des Raf-Kinase-Weges. Ein bevorzugterer Gegenstand der Erfindung sind deshalb erfindungsgemäße Derivate als Promotoren oder Inhibitoren, bevorzugt als Inhibitoren der Raf-Kinase. Ein noch bevorzugterer Gegenstand der Erfindung sind erfindungsgemäße Verbindungen als Promotoren oder Inhibitoren, bevorzugt als Inhibitoren einer oder mehrerer Raf-Kinasen, ausgewählt aus der Gruppe bestehend aus A-Raf, B-Raf und C-Raf-1. Ein besonders bevorzugter Gegenstand der Erfindung sind erfindungsgemäße Verbindungen als Promotoren oder Inhibitoren, bevorzugt als Inhibitoren von C-Raf-1.

Ein weiterer Gegenstand der vorliegenden Erfindung ist die Verwendung einer oder mehrerer erfindungsgemäßer Verbindungen bei der Behandlung und/oder Prophylaxe von Erkrankungen, bevorzugt den hier beschriebenen Erkrankungen, die durch Raf-Kinasen veruracht, vermittelt und/oder propagiert werden und insbesondere Erkrankungen, die durch Raf-Kinasen ausgewählt aus der Gruppe, bestehend aus A-Raf, B-Raf and C-Raf-1 verursacht, vermittelt und/oder propagiert werden. Gewöhnlich werden die hier besprochenen Erkrankungen in zwei Gruppen eingeteilt, in hyperproliferative und nicht hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiten und Immunschwächekrankheiten als nicht krebsartige Krankheiten angesehen, von denen Arthritis, Entzündung, immunologische Krankheiten, Autoimmunkrankheiten und Immunschwächekrankheiten gewöhnlich als nicht hyperproliferative Erkrankungen angesehen werden. In diesem Zusammenhang sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich als hyperproliferative Erkrankungen angesehen werden. Insbesondere krebsartiges Zellwachstum und insbesondere durch Raf-Kinase vermitteltes krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

In einer Vergleichsmessung wurde weiterhin gefunden, daß die Verbindungen der Formel I als PKB-Inhibitoren wirken. Diese Wirkung kann zum Beispiel durch ein Verfahren nachgewiesen werden, das von Alessi et al. EMBO L. 1996, 15, 6541-6551 beschrieben wird.

Es kann gezeigt werden, dass die erfindungsgemäßen Verbindungen in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die erfindungsgemäßen Verbindungen eignen sich darüberhinaus als Nahrungsmittelzusatzstoffe, zur Behandlung von Krankheiten und/oder Fehlfunktionen, die durch oxidative Stressbedingungen charakterisiert sind, sowie als Nahrungsmittelzusatzstoffe, ferner in kosmetischen Formulierungen als Sonnenschutzmittel.

### STAND DER TECHNIK

In der WO 92/20642 sind bis-Mono- und bicyclische Aryl- und Heteroarylverbindungen beschrieben, die Tyrosinkinase hemmen können. Chromenonderivate sind nicht offenbart.

Chromenonderivate als Bestandteile photographischer Materialien kennt man aus Patent Nr. JP 07191431 (Anmelde-Nr. JP 0347126). Die vorliegenden Verbindungen der generischen Formel I sind als Auswahlerfindung in bezug auf den genannten Stand der Technik anzusehen.

Fluorhaltige Heteroarylflavonoide mit fungizider Wirkung kennt man aus Indian Journal of Chemistry, Section B: Organic Chemistry Including Medicinal Chemistry (1987), 26B(5), 493-5

Nachfolgend sind Chromenonderivate aufgeführt, die in der Literatur beschrieben sind, jedoch nicht im Zusammenhang der Tyrosinkinase-Inhibierung:
CAS RN 477545-79-2
   1-[(4-Oxo-4H-1-benzopyran-2-yl)carbonyl]-4-phenyl-piperazin;
CAS RN 476298-55-2
   1-[(4-Oxo-4H-1-benzopyran-2-yl)carbonyl]-3,5-dimethyl-piperidin;
CAS RN 380469-63-6
   10-[(4-Oxo-4H-1-benzopyran-2-yl)carbonyl]-phenothiazin;
CAS RN 380328-67-6
   1-[(4-Oxo-4H-1-benzopyran-2-yl)carbonyl]-4-piperidincarbonsäureethylester;
CAS RN 361166-59-8
   1-[(4-Oxo-4H-1-benzopyran-2-yl)carbonyl]-1H-indol;
CAS RN 361166-57-6
   1,2,3,4-tetrahydro-2-[(4-oxo-4H-1-benzopyran-2-yl)carbonyl]-isochinolin;
CAS RN 361166-50-9
   1,2,3,4-tetrahydro-2-[(4-oxo-4H-1-benzopyran-2-yl)carbonyl]-chinolin;
CAS RN 361166-57-6
   4-[(6-Brom-4-oxo-4H-1-benzopyran-2-yl)carbonyl]-morpholin;
CAS RN 352667-41-5
   4-[(6-Chlor-4-oxo-4H-1-benzopyran-2-yl)carbonyl]-morpholin;
CAS RN 352667-39-1
   1-[(6-Brom-4-oxo-4H-1-benzopyran-2-yl)carbonyl]-pyrrolidin;
CAS RN 352667-38-0
   1-[(6-Brom-4-oxo-4H-1-benzopyran-2-yl)carbonyl]-piperidin;
CAS RN 113734-98-8
   2-[(8-Brom-6-fluor-4-oxo-4H-1-benzopyran-2-yl)carbonyl]-thiophen.

### ZUSAMMENFASSUNG DER ERFINDUNG

Die Erfindung betrifft Verbindungen der Formel I 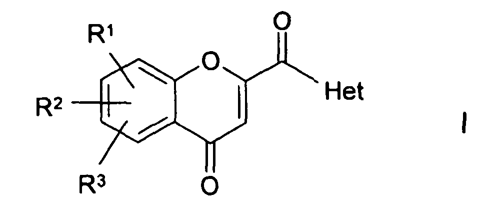 worin
- R¹: -OH oder -OA,
- R²: H, -OH, -OA, Phenoxy, -O-CO-A, -OSO₃H, -OSO₃A, -OSO₂A oder Hal,
- R³: H,
- R¹ und R²: zusammen auch Methylendioxy oder Ethylendioxy,
- Het: unsubstituiertes oder ein- oder zweifach durch Hal und/oder A substituiertes Chromen-2-on-yl, Benzothiazolyl, Thienyl, Pyridinyl, Pyrimidinyl, Pyrazinyl, Indolyl, Furyl, Pyrrolyl, Isoxazolyl, Imidazolyl, Thiazolyl, Triazolyl, Tetrazolyl, Chinolyl oder Isochinolyl,
- A: unverzweigtes oder verzweigtes Alkyl mit 1-6 C-Atomen, wobei 1-5 H-Atome durch F ersetzt sein können,
- Hal: F, Cl, Br oder I
bedeuten,
sowie ihre pharmazeutisch verwendbaren Solvate und Salze; einschließlich deren Mischungen in allen Verhältnissen.

Gegenstand der Erfindung sind die Verbindungen der Formel I und ihre Salze sowie ein Verfahren zur Herstellung von Verbindungen der Formel nach den Ansprüchen 27-29 sowie ihrer pharmazeutisch verwendbaren Solvate und salze, dadurch gekennzeichnet, daß man
a) zunächst eine Verbindung der Formel II 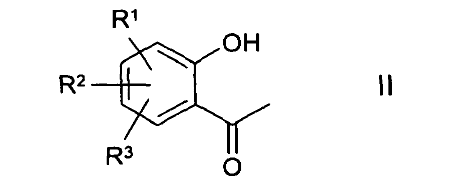 worin
   R¹, R², R³ die in Anspruch 27 angegebene Bedeutung haben, mit einer Verbindung der Formel III 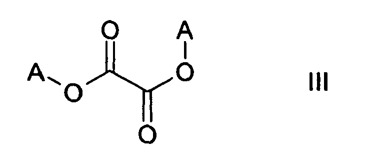 worin A Alkyl mit 1, 2, 3, 4, 5 oder 6 C-Atomen bedeutet,
   zu einer Verbindung der Formel IV 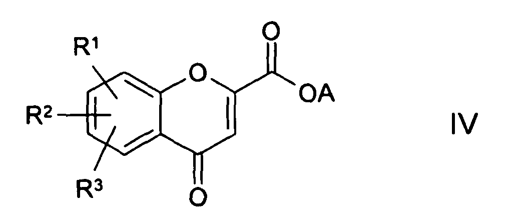 worin R¹, R², R³ die in Anspruch 27 angegebene Bedeutung haben, und A Alkyl mit 1, 2, 3, 4, 5 oder 6 C-Atomen bedeutet,
   umsetzt,
b) anschließend den Ester IV mit einer Verbindung der Formel V

   M-Het V

   worin Het die in Anspruch 27 angegebene Bedeutung hat, und M Natrium, Kalium oder Lithium bedeutet,
   zu einer Verbindung der Formel I umsetzt
   und/oder
c) eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Für alle Reste, die mehrfach auftreten, wie z.B. A, gilt, daß deren Bedeutungen unabhängig voneinander sind.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

A' bedeutet vorzugsweise Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl oder Benzyl.

OA bedeutet Alkoxy und ist vorzugsweise z.B. Methoxy, Ethoxy, Propoxy, Isopropoxy, Butoxy, Trifluormethoxy oder Cyclopentoxy.

-COA (Acyl) bedeutet vorzugsweise Acetyl, Propionyl, ferner auch Butyryl, Pentanoyl, Hexanoyl oder z.B. Benzoyl.

Hal bedeutet vorzugsweise F, Cl oder Br, aber auch I.

Ar bedeutet z.B. unsubstituiertes Phenyl, Naphthyl oder Biphenyl, weiterhin vorzugsweise z.B. durch A, Fluor, Chlor, Brom, lod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl.

Ar bedeutet ganz besonders bevorzugt Phenyl.

Het bedeutet, von den möglichen Substituenten abgesehen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Chromenyl.

Die heterocyclischen Reste können auch teilweise oder vollständig hydriert sein.

Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder - 5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder-4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet besonders bevorzugt unsubstituiertes oder ein- oder zweifach durch Hal und/oder A substituiertes Chromen-2-on-yl, Benzothiazolyl, Thienyl, Pyridinyl, Pyrimidinyl, Pyrazinyl, Indolyl, Furyl, Pyrrolyl, Isoxazolyl, Imidazolyl, Thiazolyl, Triazolyl, Tetrazolyl, Chinolyl oder Isochinolyl.

R¹ bedeutet vorzugsweise -OH, -OA, Phenoxy, -O-CO-A, -OSO₃H, -OSO₃A oder -OSO₂A, ganz besonders bevorzugt -OH oder -OA.

R² bedeutet vorzugsweise H, -OH, -OA, Phenoxy, -O-CO-A, -OSO₃H,
-OSO₃A, -OSO₂A oder Hal.
R³ bedeutet vorzugsweise H.
R¹ und R² bedeuten zusammen vorzugsweise auch Methylendioxy.

Die Verbindungen der Formel I können in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Eine bevorzugte Gruppe von Verbindungen kann durch die folgende Teilformel Ia ausgedrückt werden, die der Formel I entspricht und worin die nicht näher bezeichneten Reste die bei der Formel I angegebene Bedeutung haben, worin jedoch
in Ia
- R¹: -OH oder -OA,
- R²: H, -OH, -OA oder Hal,
- R³: H,
- R¹ und R²: zusammen auch Methylendioxy oder Ethylendioxy,
- Het: unsubstituiertes oder ein- oder zweifach durch Hal und/oder A substituiertes Benzothiazolyl, Pyridinyl, Pyrimidinyl, Pyrazinyl, Indolyl, Furyl, Pyrrolyl, Isoxazolyl, Imidazolyl oder Thiazolyl,
- A: unverzweigtes oder verzweigtes Alkyl mit 1-6 C-Atomen, wobei 1-5 H-Atome durch F ersetzt sein können,
bedeuten,
sowie ihre pharmazeutisch verwendbaren Solvate und Salze, einschließlich deren Mischungen in allen Verhältnissen.

Gegenstand der Erfindung sind insbesondere die nachstehenden Verbindungen der Formel I
6-Hydroxy-2-(1-methyl-1*H*-imidazol-2-carbonyl)-chromen-4on,
5,7-Dihydroxy-2-(1-methyl-1*H*-imidazol-2-carbonyl)-chromen-4on,
7-Hydroxy-2-(1-methyl-1*H*-imidazol-2-carbonyl)-chromen-4on,
6-(1-Methyl-1*H*-imidazol-2-carbonyl)-[1,3]dioxolo[4,5*g*]chromen-8-on,
5,7-Dihydroxy-2-(pyridin-2-carbonyl)-chromen-4-on,
6-Hydroxy-2-(pyridin-2-carbonyl)-chromen-4-on,
6-Hydroxy-2-(3,5-dichlorpyrazin-2-carbonyl)-chromen-4-on,
6-Hydroxy-2-(benzothiazolyl-2-carbonyl)-chromen-4-on,
sowie ihre pharmazeutisch verwendbaren Solvate und Salze, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II und III sind in der Regel bekannt. Sind sie neu, so können sie aber nach an sich bekannten Methoden hergestellt werden.

Verbindungen der Formel I können vorzugsweise erhalten werden, indem man Verbindungen der Formel II mit Verbindungen der Formel III zuerst zu Verbindungen der Formel IV umsetzt.

Die Umsetzung erfolgt nach Methoden, die dem Fachmann bekannt sind. Zunächst erfolgt Reaktion in einem geeigneten Alkohol in Gegenwart eines Alkali- oder Erdalkalialkoholats, z.B. in Ethanol/Natrium-ethanolat oder Methanol/Kalium-methanolat.

Grundsätzlich können auch die nachfolgenden inerten Lösungsmittel verwendet werden.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Die Cyclisierung zur Verbindung der Formel IV erfolgt säurekatalysiert, durch Zugabe geeigneter Mineralsäuren, wie z.B. Salzsäure, Phosphorsäure oder Schwefelsäure.

Reaktionszeit und Temperatur sind vorzugsweise wie oben angegeben.

Die Ester der Formel IV werden mit den Verbindungen der Formel V nach Standardmethoden in einem inerten Lösungsmittel zu den Verbindungen der Formel I umgesetzt.
Als Verbindung der Formel V wird vorzugsweise Li-Het eingesetzt. Inertes Lösungsmittel, Reaktionszeit und Temperatur sind vorzugsweise wie oben angegeben, besonders bevorzugt ist die Umsetzung in THF bei -80 bis -75 °C.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Solvate und Salze, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Gegenstand der Erfindung sind ferner Arzneimittel enthaltend mindestens eine Verbindung der Formel I und/oder ihre pharmazeutisch verwendbaren Solvate und Salze, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Solvate und Salze, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Solvate und Salze, einschließlich deren Mischungen in allen Verhältnissen,
und einer wirksamen Menge eines weiteren Arzneimittelwirkstoffs gelöst oder in lyophylisierter Form vorliegt.

### VERWENDUNG

### Einzelheiten

### I.

Gegenstand der Erfindung ist auch die Verwendung von Verbindungen der nachstehenden Formel I gemäß Anspruch 1 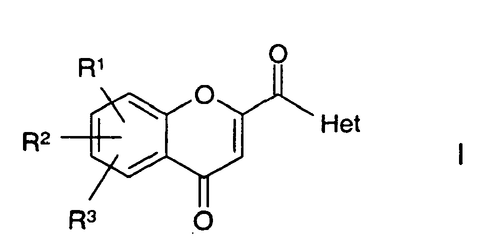 worin
- R¹: H, -OH, -OA, Phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO oder SO₂NH₂,
- R²: H, -OH, -OA, Phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO oder SO₂NH₂,
- R³: H, -OH, -OA, Phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO oder SO₂NH₂,
- R¹ und R²: zusammen auch Methylendioxy oder Ethylendioxy,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, =S, =NH, Hal, A, -(CH₂)ₒ-Ar, -(CH₂)ₒ-Cycloalkyl, -(CH₂)ₒ-OH, -(CH₂)ₒ-NH₂, NO₂, CN, -(CH₂)ₒ-COOH, -(CH₂)ₒ-COOA, -(CH₂)ₒ-CONH₂, -(CH₂)ₒ-NHCOA, NHCONH₂, -(CH₂)ₒ-NHSO₂A, CHO, COA', SO₂NH₂ und/oder S(O)ₒA substituiert sein kann,
- Ar: unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂ oder S(O)ₒA substituiertes Phenyl, Naphthyl oder Biphenyl,
- A: unverzweigtes oder verzweigtes Alkyl mit 1-10 C-Atomen, wobei 1-7 H-Atome durch F ersetzt sein können,
- A': Alkyl mit 1 bis 6 C-Atomen oder Benzyl,
- Hal: F, Cl, Br oder I,
- o: 0, 1 oder 2,
bedeuten,
sowie ihrer pharmazeutisch verwendbaren Solvate und Salze, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Hierbei sind Tyrosinkinasen und/oder Raf-Kinasen bevorzugt.

Gegenstand der Erfindung ist weiterhin die Verwendung von Verbindungen der Formel I gemäß Anspruch 1 oder 2, sowie ihrer pharmazeutisch verwendbaren Solvate und Salze, einschließlich deren Mischungen in allen Verhältnissen,
zur Herstellung eines Arzneimittels zur Behandlung von Krankheiten, die durch die Inhibierung der Tyrosinkinase durch die Verbindungen der Formel I beeinflußt werden.

Gegenstand der Erfindung ist weiterhin die Verwendung nach Anspruch 1 oder 2 von Verbindungen der Formel I gemäß Anspruch 1, sowie ihrer pharmazeutisch verwendbaren Solvate und Salze, einschließlich deren Mischungen in allen Verhältnissen, zur Herstellung eines Arzneimittels zur Behandlung von Krankheiten, die durch Raf-Kinasen verursacht, vermittelt und/oder propagiert werden wobei die Raf-Kinase aus der Gruppe bestehend aus A-Raf, B-Raf und Raf-1 ausgewählt wird.

Die Bedeutungen und bevorzugten Ausführungsführen entsprechen im allgemeinen denen wie unter **ZUSAMMENFASSUNG DER ERFINDUNG** ausgeführt.

Dementsprechend sind Gegenstand der Erfindung insbesondere die Verwendungen gemäß einem oder mehreren der Ansprüche 1 bis 26 von denjenigen Verbindungen der Formel I, in denen mindestens einer der genannten Reste eine der vorstehend angegebenen bevorzugten Bedeutungen hat. Einige bevorzugte Verwendungen von Gruppen von Verbindungen können durch die folgenden Teilformeln la bis lg ausgedrückt werden, die der Formel I entsprechen und worin die nicht näher bezeichneten Reste die bei der Formel I angegebene Bedeutung haben, worin jedoch
in Ia
- R¹: H, -OH, -OA, Phenoxy, -O-CO-A, -OSO₃H, -OSO₃A, -OSO₂A oder Hal
bedeutet;
in Ib
- R¹: H, -OH, -OA, Phenoxy, -O-CO-A, -OSO₃H, -OSO₃A, -OSO₂A oder Hal,
- R²: H, -OH, -OA, Phenoxy, -O-CO-A, -OSO₃H, -OSO₃A, -OSO₂A oder Hal,
- R³: H,
- R¹ und R²: zusammen auch Methylendioxy oder Ethylendioxy,
bedeuten;
in Ic
- R¹: -OH, -OA, Phenoxy, -O-CO-A, -OSO₃H, -OSO₃A, -OSO₂A oder Hal,
- R²: H, -OH, -OA, Phenoxy, -O-CO-A, -OSO₃H, -OSO₃A, -OSO₂A oder Hal,
- R³: H,
- R¹ und R²: zusammen auch Methylendioxy oder Ethylendioxy,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal und/oder A substituiert sein kann;
in Id
- R¹: -OH, -OA, Phenoxy, -O-CO-A, -OSO₃H, -OSO₃A oder -OSO₂A,
- R²: H, -OH, -OA, Phenoxy, -O-CO-A, -OSO₃H, -OSO₃A, -OSO₂A oder Hal,
- R³: H,
- R¹ und R²: zusammen auch Methylendioxy oder Ethylendioxy,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal und/oder A substituiert sein kann,
bedeuten;
in le
- R¹: -OH, -OA, Phenoxy, -O-CO-A, -OSO₃H, -OSO₃A, -OSO₂A oder Hal,
- R²: H, -OH, -OA, Phenoxy, -O-CO-A,
- R³: H,
- R¹ und R²: zusammen auch Methylendioxy oder Ethylendioxy,
- Het: einen ein- oder zweikernigen aromatischen Heterocyclus mit 1 bis 3 N-, O- und/oder S-Atomen, der unsubstituiert oder ein-, zwei- oder dreifach durch Hal und/oder A substituiert sein kann,
bedeuten;
in If
- R¹: -OH oder -OA,
- R²: H, -OH, -OA, Phenoxy, -O-CO-A, -OSO₃H, -OSO₃A, -OSO₂A oder Hal,
- R³: H,
- R¹ und R²: zusammen auch Methylendioxy oder Ethylendioxy,
- Het: einen ein- oder zweikernigen aromatischen Heterocyclus mit 1 bis 4 N-, O- und/oder S-Atomen, der unsubstituiert oder ein-, zwei- oder dreifach durch Hal und/oder A substituiert sein kann,
bedeuten;
in Ig
- R¹: -OH oder -OA,
- R²: H, -OH, -OA, Phenoxy, -O-CO-A, -OSO₃H, -OSO₃A, -OSO₂A oder Hal,
- R³: H,
- R¹ und R²: zusammen auch Methylendioxy oder Ethylendioxy,
- Het: unsubstituiertes oder ein- oder zweifach durch Hal und/oder A substituiertes Chromen-2-on-yl, Benzothiazolyl, Thienyl, Pyridinyl, Pyrimidinyl, Pyrazinyl, Indolyl, Furyl, Pyrrolyl, Isoxazolyl, Imidazolyl, Thiazolyl, Triazolyl, Tetrazolyl, Chinolyl oder Isochinolyl,
- A: unverzweigtes oder verzweigtes Alkyl mit 1-6 C-Atomen, wobei 1-5 H-Atome durch F ersetzt sein können,
bedeuten,
sowie ihre pharmazeutisch verwendbaren Solvate und Salze, einschließlich deren Mischungen in allen Verhältnissen.

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung von Verbindungen der Formel I nach Anspruch 1 und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom. Ebensfalls umfasst ist die Verwendung von Verbindungen der Formel I nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von Verbindungen der Formel I nach Anspruch 1 und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung von Verbindungen der Formel I nach Anspruch 1 und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer Verbindung der Formel I verabreicht. Die therapeutische Menge hängt von der jeweiligen Krankheit ab und kann vom Fachmann ohne allen großen Aufwand bestimmt werden.
Die vorliegende Erfindung umfasst auch die Verwendung von Verbindungen der Formel I nach Anspruch 1 und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.
Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die erfindungsgemäßen Verbindungen der Formel I nach Anspruch 1 können an Patienten zur Behandlung von Krebs verabreicht werden. Die vorliegenden Verbindungen hemmen die Tumorangiogenese und beeinflussen so das Wachstum von Tumoren (J. Rak et al. Cancer Research, 55:4575-4580, 1995). Die angiogenesehemmenden Eigenschaften der vorliegenden Verbindungen der Formel I nach Anspruch 1 eignen sich auch zur Behandlung bestimmter Formen von Blindheit, die mit Retina-Gefäßneubildung in Zusammenhang stehen.
Die Verbindungen der Formel I nach Anspruch 1 eignen sich auch zur Behandlung bestimmter Knochen-Pathologien wie Osteosarkom, Osteoarthritis und Rachitis, die auch unter der Bezeichnung onkogene Osteomalazie bekannt ist (Hasegawa et al., Skeletal Radiol. 28, S.41-45, 1999; Gerber et al., Nature Medicine, Bd. 5, Nr. 6, S.623-628, Juni 1999). Da der VEGF durch den in reifen Osteoklasten exprimierten KDR/Flk-1 direkt die osteoklastische Knochenresorption fördert (FEBS Let. 473:161-164 (2000); Endocrinology, 141:1667 (2000)), eignen sich die vorliegenden Verbindungen auch zur Behandlung und Vorbeugung von Leiden, die mit Knochenresorption in Zusammenhang stehen, wie Osteoporose und Morbus Paget.
Die Verbindungen können dadurch, dass sie zerebrale Ödeme, Gewebeschädigung und ischämiebedingte Reperfusionsverletzungen reduzieren, auch zur Verringerung oder Vorbeugung von Gewebeschäden, die nach zerebralen ischämischen Ereignissen wie Gehirnschlag auftreten, verwendet werden (Drug News Perspect 11:265-270 (1998); J. Clin. Invest. 104:1613-1620 (1999)).

Die Verbindungen der Formel I nach Anspruch 1 eignen sich zur Herstellung eines Arzneimittels zur Behandlung von Krankheiten, die durch Raf-Kinasen verursacht, vermittelt und/oder propagiert werden, wobei die Raf-Kinase aus der Gruppe bestehend aus A-Raf, B-Raf und Raf-1 ausgewählt wird.
Bevorzugt ist die Verwendung zur Behandlung von Erkrankungen, vorzugsweise aus der Gruppe der hyperproliferativen und nicht hyperproliferativen Erkrankungen.

Hierbei handelt es sich um Krebserkrankungen oder nicht krebsartige Erkrankungen.
Die nicht krebsartigen Erkrankungen sind ausgewählt aus der Gruppe bestehend aus Psoriasis, Arthritis, Entzündungen, Endometriose, Vernarbung, gutartiger Prostatahyperplasie, immunologischer Krankheiten, Autoimmunkrankheiten und Immunschwächekrankheiten.

Die krebsartigen Erkrankungen sind ausgewählt aus der Gruppe bestehend aus Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, chronischer Leukämie und akuter Leukämie.

Die erfindungsgemäßen Verbindungen können an Säugetiere, vorzugsweise den Menschen, entweder allein oder vorzugsweise in Kombination mit pharmazeutisch unbedenklichen Trägern oder Streckmitteln, gegebenenfalls mit bekannten Hilfsstoffen wie Alaun, in einer pharmazeutischen Zusammensetzung wie in der pharmazeutischen Praxis üblich verabreicht werden. Die Verbindungen können oral oder parenteral verabreicht werden, darunter auf dem intravenösen, intramuskulären, intreperitonealen, subkutanen, rektalen und topischen Verabreichungsweg.
Bei der oralen Verwendung einer erfindungsgemäßen chemotherapeutischen Verbindung kann die gewählte Verbindung zum Beispiel in Form von Tabletten oder Kapseln oder als wässrige Lösung oder Suspension verabreicht werden. Bei Oraltabletten zählen zu den üblicherweise verwendeten Trägern Laktose und Maisstärke, und es werden üblicherweise Gleitmittel wie Magnesiumstearat zugegeben. Bei der oralen Verabreichung in Kapselform zählen Laktose und getrocknetete Maisstärke zu geeigneten Streckmitteln. Sind wässrige Suspensionen zur oralen Verwendung erforderlich, so wird der Wirkstoff mit Emulgatoren und Suspendiermitteln zusammengegeben. Gewünschtenfalls können bestimmte Süßstoffe und/oder Aromastoffe zugegeben werden. Für die intramuskuläre, intraperitoneale, subkutane und intravenöse Verwendung werden üblichwerweise sterile Lösungen des Wirkstoffs hergestellt, und der pH-Wert der Lösungen sollte auf geeignete Weise eingestellt und gepuffert werden. Bei der intravenösen Verwendung sollte die Gesamtkonzentration an gelösten Substanzen so eingestellt werden, dass das Präparat isotonisch wird. Die genaue Dosis für den jeweiligen Patienten hängt jedoch von einer Reihe von Faktoren ab, beispielsweise von der Wirksamkeit der jeweiligen verwendeten Verbindungen, vom Alter, Körpergewicht, allgemeinen Gesundheitszustand, Geschlecht, von der Ernährung, von Verabreichungszeitpunkt und -weg, von der Ausscheidungsrate, dem Verabreichungstyp, der zu verabreichenden Arzneiform, der Medikamentenkombination und der Schwere der Krankheit, gegen die die Therapie eingesetzt wird. Die jeweilige therapeutisch wirksame Dosis für den jeweiligen Patienten kann leicht durch Routineversuche bestimmt werden, zum Beispiel durch den Arzt, der zu dieser therapeutischen Behandlung rät bzw. sie begleitet.

Die erfindungsgemäßen Substanzen werden in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht.

Die erfindungsgemäßen Verbindungen können auch gemeinsam mit anderen gut bekannten Therapeutika, die aufgrund ihrer jeweiligen Eignung für das behandelte Leiden ausgewählt werden, verabreicht werden. So wären zum Beispiel bei Knochenleiden Kombinationen günstig, die antiresorptiv wirkende Bisphosphonate, wie Alendronat und Risedronat, Integrinblocker (wie sie weiter unten definiert werden), wie avβ3-Antagonisten, bei der Hormontherapie verwendetete konjugierte Östrogene wie Prempro®, Premarin® und Endometrion®; selektive Östrogenrezeptormodulatoren (SERMs) wie Raloxifen, Droloxifen, CP-336,156 (Pfizer) und Lasofoxifen, Kathepsin-K-Hemmer und ATP-Protonenpumpenhemmer enthalten.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diaminplatin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Hemmer sind zum Beispiel Topotecan, Hycaptamin, Irinotecan, Rubitecan, 6-Ethoxypropionyl-3',4'-O-exo-benzylidenchartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethylamino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-mannoheptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehydthiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).
VEGF-Rezeptorkinase-Assay
Die VEGF-Rezeptorkinaseaktivität wird durch Einbau von radioaktiv markiertem Phosphat in 4:1 Polyglutaminsäure/Tyrosin-Substrat (pEY) bestimmt. Das phosphorylierte pEY-Produkt wird auf einer Filtermembran festgehalten, und der Einbau des radioaktiv markierten Phosphats wird durch Szintillationszählung quantitativ bestimmt.

### MATERIALIEN

### VEGF-Rezeptorkinase

Die intrazelluläre-Tyrosinkinase-Domänen des menschlichen KDR (Terman, B. I. et al. Oncogene (1991) Bd. 6, S. 1677-1683.) und Flt-1 (Shibuya, M. et al. Oncogene (1990) Bd. 5, S. 519-524) wurden als Glutathion-S-transferase (GST)-Genfusionsproteine kloniert. Dies geschah durch Klonieren der Zytoplasma-Domäne der KDR-Kinase als leserastergerechte Fusion am Carboxy-Terminus des GST-Gens. Die löslichen rekombinanten GST-Kinasedomäne-Fusionsproteine wurden in *Spodoptera frugiperda* (Sf21) Insektenzellen (Invitrogen) unter Verwendung eines Baculovirus-Expressionsvektors (pAcG2T, Pharmingen) exprimiert.

### Lysepuffer

50 mM Tris pH 7,4, 0,5 M NaCl, 5 mM DTT, 1 mM EDTA, 0,5% Triton X-100, 10% Glycerin, je 10 mg/ml Leupeptin, Pepstatin und Aprotinin sowie 1 mM Phenylmethylsulfonylfluorid (alle von Sigma).

### Waschpuffer

50 mM Tris pH 7,4, 0,5 M NaCl, 5 mM DTT, 1 mM EDTA, 0.05% Triton X-100, 10% Glycerin, je 10 mg/ml Leupeptin, Pepstatin und Aprotinin sowie 1 mM Phenylmethylsulfonylfluorid.

### Dialysepuffer

50 mM Tris pH 7,4, 0,5 M NaCl, 5 mM DTT, 1 mM EDTA, 0.05% Triton X-100, 50% Glycerin, je 10 mg/ml Leupeptin, Pepstatin und Aprotinin sowie 1 mM Phenylmethylsulfonylfluorid.

### 10x Reaktionspuffer

200 mM Tris, pH 7,4, 1,0 M NaCl, 50 mM MnCl₂, 10 mM DTT und 5 mg/ml Rinderserumalbumin [bovine serum albumin = BSA] (Sigma).

### Enzymverdünnungspuffer

50 mM Tris, pH 7,4, 0,1 M NaCl, 1 mM DTT, 10% Glycerin, 100 mg/ml BSA.

### 10x Substrat

750 µg/ml Poly(glutaminsäure/Tyrosin; 4:1) (Sigma).

### Stopp-Lösung

30% Trichloressigsäure, 0,2 M Natriumpyrophosphat (beide von Fisher). Waschlösung
15% Trichloressigsäure, 0,2 M Natriumpyrophosphat.

### Filterplatten

### Millipore #MAFC NOB, GF/C 96-Well-Glasfaserplatte.

### Verfahren A - Proteinaufreinigung

1. Die Sf21-Zellen wurden mit dem rekombinanten Virus bei einer m.o.i. (Multiplizität der Infektion) von 5 Viruspartikeln/Zelle infiziert und 48 Stunden lang bei 27°C gezüchtet.
2. Alle Schritte wurden bei 4°C durchgeführt. Die infizierten Zellen wurden durch Zentrifugieren bei 1000xg geerntet und 30 Minuten bei 4°C mit 1/10 Volumen Lysepuffer lysiert und anschließend 1 Stunde lang bei 100.000xg zentrifugiert. Der Überstand wurde dann über eine mit Lysepuffer äquilibrierte Glutathion-Sepharose-Säure (Pharmacia) gegeben und mit 5 Volumina des gleichen Puffers und anschließend 5 Volumina Waschpuffer gewaschen. Das rekombinante GST-KDR-Protein wurde mit Waschpuffer/10 mM reduziertem Glutathion (Sigma) eluiert und gegen Dialysepuffer dialysiert.

### Verfahren B - VEGF-Rezeptorkinase-Assay

1. Assay mit 5 µl Hemmstoff oder Kontrolle in 50% DMSO versetzen.
2. Mit 35 µl Reaktionsmischung, die 5 µl 10x Reaktionspuffer, 5 µl 25 mM ATP/10 µCi[³³ P]ATP (Amersham) und 5 µl 10x Substrat enthält, versetzen.
3. Reaktion durch Zugabe von 10 µl KDR (25 nM) in Enzymverdünnungspuffer starten.
4. Mischen und 15 Minuten lang bei Raumtemperatur inkubieren.
5. Reaktion durch Zugabe von 50 µl Stopp-Lösung stoppen.
6. 15 Minuten lang bei 4°C inkubieren.
7. 90-µl-Aliquot auf Filterplatte überführen.
8. Absaugen und 3 Mal mit Waschlösung waschen.
9. 30 µl Szintillations-Cocktail zugeben, Platte verschließen und in einem Szintillations-Zähler Typ Wallac Microbeta zählen.

Mitogenese-Assay an menschlichen Nabelschnurvenenendothelzellen

Die Expression von VEGF-Rezeptoren, die mitogene Reaktionen auf den Wachstumsfaktor vermitteln, ist größtenteils auf Gefäßendothelzellen beschränkt. Kultivierte menschliche Nabelschnurvenenendothelzellen (HUVECs) proliferieren als Reaktion auf Behandlung mit VEGF und können als Assaysystem zur quantitativen Bestimmung der Auswirkungen von KDR-Kinasehemmern auf die Stimulation des VEGF verwendet werden. In dem beschriebenen Assay werden Einzelzellschichten von HUVECs im Ruhezustand 2 Stunden vor der Zugabe von VEGF oder "basic fibroblast growth factor" (bFGF) mit dem Konstituens oder der Testverbindung behandelt. Die mitogene Reaktion auf VEGF oder bFGF wird durch Messung des Einbaus von [³H]Thymidin in die Zell-DNA bestimmt.

### Materialien

### HUVECs

Als Primärkulturisolate tiefgefrorene HUVECs werden von Clonetics Corp bezogen. Die Zellen werden im Endothel-Wachstumsmedium (Endothelial Growth Medium = EGM; Clonetics) erhalten und in der 3. - 7. Passage für die Mitogenitätsassays verwendet.

### Kulturplatten

### NUNCLON 96-Well-Polystyrol-Gewebekulturplattten (NUNC #167008). Assay-Medium

Nach Dulbecco modifiziertes Eagle-Medium mit 1 g/ml Glucose (DMEM mit niedrigem Glucosegehalt; Mediatech) plus 10% (v/v) fötales Rinderserum (Clonetics).

### Testverbindungen

Mit den Arbeitsstammlösungen der Testverbindungen wird mit 100% Dimethylsulfoxid (DMSO) solange eine Reihenverdünnung durchgeführt, bis ihre Konzentrationen um das 400-fache höher als die gewünschte Endkonzentration sind. Die letzten Verdünnungen (Konzentration 1 x) werden unmittelbar vor Zugabe zu den Zellen mit Assay-Medium hergestellt.

### 10x Wachstumsfaktoren

Lösungen des menschlichen VEGF 165 (500 ng/ml; R&D Systems) und bFGF (10 ng/ml; R&D Systems) werden mit Assay-Medium hergestellt.

### 10× [³H]-Thymidin

[Methyl-³H]-Thymidin (20 Ci/mmol; Dupont-NEN) wird mit DMEM-Medium mit niedrigem Glucosegehalt auf 80 µCi/ml verdünnt.

### Zellwaschmedium

Hank's balanced salt solution (Mediatech) mit 1 mg/ml Rinderserumalbumin (Boehringer-Mannheim).

### Zell-Lyse-Lösung

1 N NaOH, 2% (w/v) Na₂CO₃.

### Verfahren 1

In EGM gehaltene HUVEC-Einzelzellschichten werden durch Trypsinbehandlung geerntet und in einer Dichte von 4000 Zellen pro 100 µl Assay-Medium pro Näpfchen in 96-Well-Platten überimpft. Das Wachstum der Zellen wird 24 Stunden bei 37°C in einer 5% CO₂ enthaltenden feuchten Atmosphäre gestoppt.

### Verfahren 2

Das Wachstumsstoppmedium wird durch 100 µl Assay-Medium ersetzt, das entweder das Konstituens (0,25% [v/v] DMSO) oder die erwünschte Endkonzentration der Testverbindung enthält. Alle Bestimmungen werden in dreifacher Wiederholung durchgeführt. Die Zellen werden dann 2 Stunden bei 37°C/5% CO₂ inkubiert, so dass die Testverbindungen in die Zellen eindringen können.

### Verfahren 3

Nach 2-stündiger Vorbehandlung werden die Zellen durch Zugabe von 10 µl Assay-Medium, 10x VEGF-Lösung oder 10x bFGF-Lösung pro Näpfchen stimuliert. Die Zellen werden dann bei 37°C/5% CO₂ inkubiert.

### Verfahren 4

Nach 24 Stunden in Anwesenheit der Wachstumsfaktoren wird mit 10x [³H]-Thymidin (10 µl/well) versetzt.

### Verfahren 5

Drei Tage nach dem Versetzen mit [³H]-Thymidin wird das Medium abgesaugt und die Zellen werden zweimal mit Zellwaschmedium gewaschen (400 µl/well, anschließend 200 µl/well). Die gewaschenen, adhärenten Zellen werden dann durch Zugabe von Zell-Lyse-Lösung (100 µl/well) und 30-minutiges Erwärmen auf 37°C solubilisiert. Die Zell-Lysate werden in 7-ml-Szintillationsrährchen aus Glas, die 150 µl Wasser enthalten, überführt. Man versetzt mit dem Szintillations-Cocktail (5 ml/Röhrchen), und die mit den Zellen assoziierte Radioaktivität wird flüssigkeitsszintillationsspektroskopisch bestimmt.
Gemäß diesen Assays stellen die Verbindungen der Formel I VEGF-Hemmer dar und eignen sich daher zur Hemmung der Angiogenese, wie bei der Behandlung von Augenkrankheiten, z.B. diabetischer Retinopathie, und zur Behandlung von Karzinomen, z.B. festen Tumoren. Die vorliegenden Verbindungen hemmen die VEGF-stimulierte Mitogenese von kultivierten menschlichen Gefäßendothelzellen mit HK50-Werten von 0,01-5,0 µM. Diese Verbindungen sind im Vergleich zu verwandten Tyrosinkinasen (z.B. FGFR1 sowie Src-Familie; zur Beziehung zwischen Src-Kinasen und VEGFR-Kinasen siehe Eliceiri et al., Molecular Cell, Bd. 4, S.915-924, Dezember 1999) auch selektiv.

### II.

Gegenstand der Erfindung ist weiterhin die Verwendung von Verbindungen der Formel I und/oder ihre physiologisch unbedenklichen Salze und Solvate zur Herstellung eines Arzneimittels zur Behandlung von Krankheiten und/oder Fehlfunktionen, die durch oxidative Stressbedingungen charakterisiert sind, bei einem Säugetier, das einer derartigen Behandlung bedarf, wobei man dem Säugetier, das einer derartigen Behandlung bedarf, eine therapeutisch wirksame Menge einer Verbindung nach Anspruch 1 oral verabreicht.

Bei den Krankheiten und/oder Fehlfunktionen handelt es sich insbesondere um Gedächtnisverlust und um neurodegenerative Erkrankungen handelt.

Die erfindungsgemäßen Verbindungen finden daher Verwendung zur Neuroprotektion.

Die Verwendung von Isoquercetin und Ascorbinsäure zu entsprechenden Zwecken ist in der WO 00/54754 beschrieben.

Gegenstand der Erfindung ist weiterhin die Verwendung der erfindungsgemäßen Verbindungen und/oder ihre physiologisch unbedenklichen Salze und Solvate als Nahrungsmittelzusatzstoffe.

Gegenstand der Erfindung ist weiterhin eine Zusammensetzung, enthaltend Ascorbinsäure, Ascorbat oder ein Ascorbinsäurederivat und mindestens eine erfindungsgemäße Verbindung und/oder ihr physiologisch unbedenkliches Salz und Solvat.

### III.

Die erfindungsgemäßen Verbindungen weisen als Flavonoidderivate antioxidative Eigenschaften auf.

Die Verwendung von Ectoinderivaten mit Antioxidationsmitteln zum Schutz von Stressproteinen der Haut in topischen Formulierungen ist z.B. in der WO 01/72263 beschrieben.

Gegenstand der Erfindung ist daher die Verwendung der erfindungsgemäßen Verbindungen und/oder ihre physiologisch unbedenklichen Salze und Solvate in kosmetischen Formulierungen, vorzugsweise in Form einer topischen Formulierung.

Gegenstand der Erfindung ist weiterhin die Verwendung der erfindungsgemäßen Verbindungen und/oder ihre physiologisch unbedenklichen Salze und Solvate zum Schutz der Streßproteine der Haut, vorzugsweise in Form einer topischen Formulierung.

Die Herstellung der topischen Zusammensetzung erfolgt, indem mindestens eine der erfindungsgemäß verwendeten Verbindungen, gegebenenfalls mit Hilfs- und/oder Trägerstoffen, in eine geeignete Formulierungsform gebracht werden. Die Hilfs- und Trägerstoffe stammen aus der Gruppe der Trägermittel, Konservierungsstoffe und anderer üblicher Hilfsstoffe.

Die topischen Zusammensetzungen auf der Grundlage mindestens einer erfindungsgemäß verwendeten Verbindung wird äußerlich auf der Haut oder den Hautadnexen angewendet.

Als Anwendungsform seien z.B. genannt, Lösungen, Suspensionen, Emulsionen, Pasten, Salben, Gele, Cremes, Lotionen, Puder, Seifen, tensidhaltige Reinigungspräparate, Öle und Sprays. Zusätzlich zu einer oder mehreren erfindungsgemäß verwendeten Verbindungen werden der Zusammensetzung beliebige übliche Trägerstoffe, Hilfsstoffe und gegebenenfalls weitere Wirkstoffe zugesetzt.

Bevorzugte Hilfsstoffe stammen aus der Gruppe der Konservierungsstoffe, Antioxidantien, Stabilisatoren, Lösungsvermittler, Vitamine, Färbemittel und Geruchsverbesserer. Salben, Pasten, Cremes und Gele können neben einer oder mehreren erfindungsgemäß verwendeten Verbindungen die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben einer oder mehreren erfindungsgemäß verwendeten Verbindungen die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether.

Lösungen und Emulsionen können neben einer oder mehreren erfindungsgemäß verwendeten Verbindungen die üblichen Trägerstoffe, wie Lösungsmittel, Lösungsvermittler und Emulgatoren, z.B. Wasser, Ethanoi, Isopropanol, Ethylcarbonat, Ethylacetat, Benzylalkohol, Benzylbenzoat, Propylenglykol, 1,3-Butylglykol, Öle, insbesondere Baumwollsaatöle, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe, enthalten.

Suspensionen können neben einer oder mehreren erfindungsgemäß verwendeten Verbindungen die üblichen Trägerstoffe, wie flüssige Verdünnungsmittel, z. B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe, enthalten.

Seifen können neben einer oder mehreren erfindungsgemäß verwendeten Verbindungen die üblichen Trägerstoffe, wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe, enthalten.

Tensidhaltige Reinigungsprodukte können neben einer oder mehreren erfindungsgemäß verwendeten Verbindungen die üblichen Trägerstoffe, wie Salze von Fettalkoholsulfaten, Fettalkoholethersulfaten, Sulfobernsteinsäurehalbestern, Fettsäureeiweißhydrolysaten, Isothionaten, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe, enthalten.

Gesichts- und Körperöle können neben einer oder mehreren erfindungsgemäß verwendeten Verbindungen die üblichen Trägerstoffe, wie synthetische Öle, wie Fettsäureester, Fettalkohole, Silikonöle, natürliche Öle, wie Pflanzenöle und ölige Pflanzenauszüge, Paraffinöle, Lanolinöle oder Gemische dieser Stoffe, enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun- Präparate.

Mindestens eine erfindungsgemäß verwendete Verbindung liegt in der topischen Zusammensetzung in einer Menge von vorzugsweise 0,0001 bis 50 Gew.-%, besonders bevorzugt 0,001 bis 10 Gew.-%, insbesondere bevorzugt 0, 1 bis 1 Gew.-%, bezogen auf die Zusammensetzung, vor.

### Test zur Untersuchung der Radikalfängereigenschaften bzw. der antioxidativen Wirkung

Der Assay dient zum Screenen der antioxidativen oder Radikalfänger-Eigenschaft einer Substanz oder eines Extrakts. Um diese Eigenschaft zu ermitteln, läßt man die Substanz mit dem stabilen Radikal DPPH* (2,2-Diphenyl-1-picrylhydrazyl Hydrat) in ethanolischer Lösung reagieren. Die Reduktion von DPPH* wird über die Abnahme der Extinktion bei der charakteristischen Wellenlänge des Radikals verfolgt. In seiner radikalischen Form absorbiert DPPH* bei 515 nm, bei der Reduktion durch ein Antioxidans (AOX) nimmt die Extinktion ab. Für jedes Antioxidans werden unterschiedliche Konzentrationen untersucht (ausgedrückt als das Verhältnis von Mol Antioxidans / Mol DPPH*). Die Abnahme der Ektinktion bei 515 nm wird nach 1 Sekunde, 2 Minuten, 10 Minuten, und dann alle 10 Minuten bestimmt, bis die Extinktion konstant bleibt. Die exakte Anfangskonzentration von DPPH* wird mit Hilfe des Extinktionskoeffizienten bestimmt. Bei jeder Antioxidanskonzentration wird die verbliebene DPPH*-Konzentration als Prozent der Ausgangskonzentration ermittelt und gegen das molare Verhältnis von Antioxidans mit DPPH* aufgetragen. Die antiradikalische Aktivität wird definiert als der Anteil des Antioxidans, der die DPPH Konzentration auf 50 Prozent der Anfangsmenge senkt (Efficient Concentration = EC₅₀). Je kleiner dieser Wert ist, dest größer ist die Aktivität gegen Radikale. Das Reaktionsverhalten der einzelnen Antioxidantien ist sehr unterschiedlich. Man kann zwischen schnell-, mittel- und langsam reagierenden Substanzen unterscheiden, das Erreichen des steady-state kann zwischen 30 Sekunden und 12 Stunden liegen.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-Ionisation) M⁺ FAB (Fast Atom Bombardment) (M+H)⁺ ESI (Electrospray lonization) (M+H)⁺ (wenn nichts anderes angegeben)

### Beispiel 1

### Herstellung von 6-Hydroxy-2-(1-methyl-1H-imidazol-2-carbonyl)-chromen-4-on

1.1 3,8 g Natrium werden portionsweise unter Rühren in 300 ml Ethanol gelöst. Anschließend wird eine Lösung von 5,0 g 2,5-Dihydroxyacetophenon und 17,8 ml Oxalsäurediethylester in 25 ml Ethanol zugetropft. Danach wird das Reaktionsgemisch 3 Stunden auf 80° erhitzt. Man kühlt auf Raumtemperatur ab und tropft 10 ml 32 %ige HCl zu. Man erhitzt 30 Minuten bei 90°, kühlt ab, und entfernt das Lösungsmittel. Nach üblicher Aufarbeitung erhält man 5,6 g 6-Hydroxy-2-ethoxycarbonyl-chromen-4-on ("AA").

1.2 Zu einer auf -78° abgekühlten Lösung von 0,3 ml 1-Methyl-1 H-imidazol in 5 ml THF gibt man unter N₂-Atmosphäre 2,4 ml einer 1,6 M n-BuLi-Lösung und rührt 30 Minuten nach. Anschließend gibt man eine Lösung von 300 mg "AA" in 5 ml THF zu und rührt 1 Stunde nach. Man arbeitet wie üblich auf und erhält 303 mg 6-Hydroxy-2-(1-methyl-1H-imidazol-2-carbonyl)-chromen-4-on.
¹H NMR (DMSO-d6) δ 4.02 (s, 3H), 7.32 (m, 3H), 7.59 (s, 1H), 7.62 (dd, *J*= 9.6, 2.0 Hz, 1H), 7.74 (s, 1H), 10.21(s,1H).
¹³C NMR (DMSO-d6) δ 36.1, 107.2, 115,3, 120.3, 124.2, 124.6, 129.7, 129.9, 141.1, 149.2, 155.2, 156.0, 174.8, 177.4.
EI MS (*m*/*z*) 267 (M⁺), 239, 211
UV-Vis (ⁱPrOH): λₘₐₓ. (1g. ε):

Analog erhält man nachstehende Verbindungen
6-Hydroxy-2-(1-methyl-1*H*-imidazol-2-carbonyl)-chromen-4on,
5,7-Dihydroxy-2-(1-methyl-1*H*-imidazol-2-carbonyl)-chromen-4on,
7-Hydroxy-2-(1-methyl-1*H*-imidazol-2-carbonyl)-chromen-4on,
6-(1-Methyl-1*H*-imidazol-2-carbonyl)-[1,3]dioxolo[4,5*g*]chromen-8-on,
5,7-Dihydroxy-2-(pyridin-2-carbonyl)-chromen-4-on,
6-Hydroxy-2-(pyridin-2-carbonyl)-chromen-4-on,
6-Hydroxy-2-(3,5-dichlorpyrazin-2-carbonyl)-chromen-4-on,
6-Hydroxy-2-(benzothiazolyl-2-carbonyl)-chromen-4-on.

### Pharmakologische Testergebnisse

| | Inhibierung von PKB IC₅₀ (µMol) | Inhibierung von RAF IC₅₀ (µMol) | Inhibierung von Tie2 IC₅₀ (µMol) |
|---|---|---|---|
| 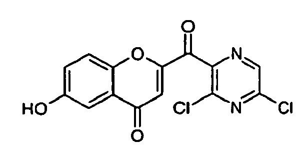 | 9,4 | > 1.0 | >10 |
| 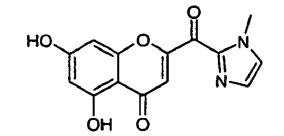 | >10 | >1.0 | >10 |
| 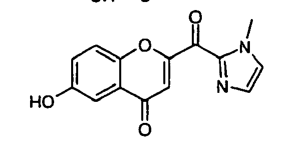 | >10 | >1.0 | >10 |
| 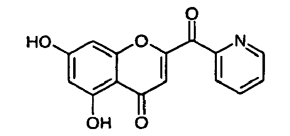 | >10 | >1.0 | >10 |
| 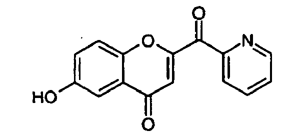 | >10 | >1.0 | >10 |
| 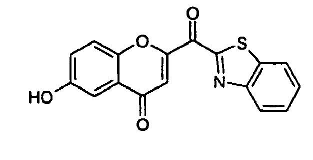 | >10 | >1.0 | >10 |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I , 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verwendung von Verbindungen der Formel I 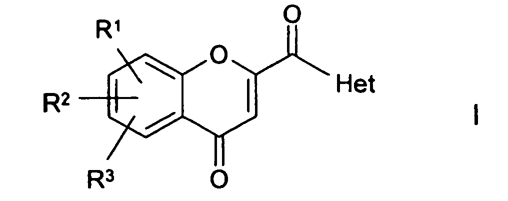 worin
R¹ H, -OH, -OA, Phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO oder SO₂NH₂,
R² H, -OH, -OA, Phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO oder SO₂NH₂,
R³ H, -OH, -OA, Phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO oder SO₂NH₂,
R¹ und R² zusammen auch Methylendioxy oder Ethylendioxy,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, =S, =NH, Hal, A, - (CH₂)ₒ-Ar, -(CH₂)ₒ-Cycloalkyl, -(CH₂)ₒ-OH, -(CH₂)ₒ-NH₂, NO₂, CN, -(CH₂)ₒ-COOH, -(CH₂)ₒ-COOA, -(CH₂)ₒ-CONH₂, -(CH₂)ₒ-NHCOA, NHCONH₂, -(CH₂)ₒ-NHSO₂A, CHO, COA', SO₂NH₂ und/oder S(O)ₒA substituiert sein kann,
Ar unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂ oder S(O)ₒA substituiertes Phenyl, Naphthyl oder Biphenyl,
A unverzweigtes oder verzweigtes Alkyl mit 1-10 C-Atomen, wobei 1-7 H-Atome durch F ersetzt sein können,
A' Alkyl mit 1 bis 6 C-Atomen oder Benzyl,
Hal F, Cl, Br oder I,
o 0, 1 oder 2,
bedeuten,
sowie ihrer pharmazeutisch verwendbaren Solvate und Salze, einschließlich deren Mischungen in allen Verhältnissen,
zur Herstellung eines Arzneimittels zur Behandlung von Krankheiten, bei denen die Hemmung, Regulierung und/oder Modulation der Signaltransduktion von Kinasen eine Rolle spielt.

2. Verwendung nach Anspruch 1, wobei die Kinasen ausgewählt sind aus der Gruppe der
Tyrosinkinasen und/oder Raf-Kinasen.

3. Verwendung nach Anspruch 1 oder 2 von Verbindungen der Formel I gemäß Anspruch 1, sowie ihrer pharmazeutisch verwendbaren Solvate und Salze, einschließlich deren Mischungen in allen Verhältnissen,
zur Herstellung eines Arzneimittels zur Behandlung von Krankheiten, die durch Inhibierung der Tyrosinkinasen durch die Verbindungen der Formel I beeinflußt werden.

4. Verwendung nach Anspruch 3, wobei die zu behandelnde Krankheit ein fester Tumor ist.

5. Verwendung nach Anspruch 4, wobei der feste Tumor aus der Gruppe Gehirntumor, Tumor des Urogenitaltrakts, Tumor des lymphatischen Systems, Magentumor, Kehlkopftumor und Lungentumor stammt.

6. Verwendung nach Anspruch 5, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom stammt.

7. Verwendung nach Anspruch 3 zur Behandlung einer Krankheit, an der Angiogenese beteiligt ist.

8. Verwendung nach Anspruch 7, wobei es sich bei der Krankheit um eine Augenkrankheit handelt.

9. Verwendung nach Anspruch 3 zur Behandlung von Retina-Vaskularisierung, diabetischer Retinopathie, altersbedingter Makula-Degeneration und/oder Entzündungskrankheiten.

10. Verwendung nach Anspruch 9, wobei die Entzündungskrankheit aus der Gruppe rheumatoide Arthritis, Schuppenflechte, Kontaktdermatitis und Spät-Typ der Überempfindlichkeitsreaktion stammt.

11. Verwendung nach Anspruch 3 zur Behandlung von Knochen-Pathologien, wobei die Knochenpathologie aus der Gruppe Osteosarkom, Osteoarthritis und Rachitis stammt.

12. Verwendung nach Anspruch 3 von Verbindungen der Formel I und/oder ihrer physiologisch unbedenklichen Salze und Solvate zur Herstellung eines Arzneimittels zur Behandlung von festen Tumoren, wobei eine therapeutisch wirksame Menge einer Verbindung nach Anspruch 1 in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

13. Verwendung nach Anspruch 3 von Verbindungen der Formel I und/oder ihrer physiologisch unbedenklichen Salze und Solvate zur Herstellung eines Arzneimittels zur Behandlung von festen Tumoren wobei eine therapeutisch wirksame Menge einer Verbindung nach Anspruch 1 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

14. Verwendung nach Anspruch 1 oder 2 von Verbindungen der Formel I gemäß Anspruch 1, sowie ihrer pharmazeutisch verwendbaren Solvate und Salze, einschließlich deren Mischungen in allen Verhältnissen,
zur Herstellung eines Arzneimittels zur Behandlung von Krankheiten, die durch Raf-Kinasen verursacht, vermittelt und/oder propagiert werden.

15. Verwendung nach Anspruch 14, wobei die Raf-Kinase aus der Gruppe bestehend aus A-Raf, B-Raf und Raf-1 ausgewählt wird.

16. Verwendung nach Anspruch 14, wobei die Erkrankungen ausgewählt sind aus der Gruppe der hyperproliferativen und nicht hyperproliferativen Erkrankungen.

17. Verwendung nach Anspruch 14 oder 16, wobei die Erkrankung Krebs ist.

18. Verwendung nach Anspruch 14 oder 16, wobei die Erkrankung nicht krebsartig ist.

19. Verwendung nach Anspruch 14, 16 oder 18, wobei die nicht krebsartigen Erkrankungen ausgewählt sind aus der Gruppe bestehend aus Psoriasis, Arthritis, Entzündungen, Endometriose, Vernarbung, gutartiger Prostatahyperplasie, immunologischer Krankheiten, Autoimmunkrankheiten und Immunschwächekrankheiten.

20. Verwendung nach einem der Ansprüche 14, 16 oder 17, wobei die Erkrankungen ausgewählt sind aus der Gruppe bestehend aus Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, chronischer Leukämie und akuter Leukämie.

21. Verwendung gemäß einem oder mehreren der Ansprüche 1 bis 20 von Verbindungen der Formel I gemäß Anspruch 1
worin
R¹ H, -OH, -OA, Phenoxy, -O-CO-A, -OSO₃H, -OSO₃A, -OSO₂A oder Hal
bedeutet,
sowie ihrer pharmazeutisch verwendbaren Solvate und Salze, einschließlich deren Mischungen in allen Verhältnissen.

22. Verwendung gemäß einem oder mehreren der Ansprüche 1 bis 21 von Verbindungen der Formel I gemäß Anspruch 1
worin
R¹ H, -OH, -OA, Phenoxy, -O-CO-A, -OSO₃H, -OSO₃A, -OSO₂A oder Hal,
R² H, -OH, -OA, Phenoxy, -O-CO-A, -OSO₃H, -OSO₃A, -OSO₂A oder Hal,
R³ H,
R¹ und R² zusammen auch Methylendioxy oder Ethylendioxy,
bedeuten,
sowie ihrer pharmazeutisch verwendbaren Solvate und Salze, einschließlich deren Mischungen in allen Verhältnissen.

23. Verwendung gemäß einem oder mehreren der Ansprüche 1 bis 22 von Verbindungen der Formel I gemäß Anspruch 1
worin
R¹ -OH, -OA, Phenoxy, -O-CO-A, -OSO₃H, -OSO₃A, -OSO₂A oder Hal,
R² H, -OH, -OA, Phenoxy, -O-CO-A, -OSO₃H, -OSO₃A, -OSO₂A oder Hal,
R³ H,
R¹ und R² zusammen auch Methylendioxy oder Ethylendioxy,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal und/oder A substituiert sein kann,
bedeuten,
sowie ihrer pharmazeutisch verwendbaren Solvate und Salze, einschließlich deren Mischungen in allen Verhältnissen.

24. Verwendung gemäß einem oder mehreren der Ansprüche 1 bis 23 von Verbindungen der Formel I gemäß Anspruch 1
worin
R¹ -OH, -OA, Phenoxy, -O-CO-A, -OSO₃H, -OSO₃A, -OSO₂A oder Hal,
R² H, -OH, -OA, Phenoxy, -O-CO-A, -OSO₃H, -OSO₃A, -OSO₂A oder Hal,
R³ H,
R¹ und R² zusammen auch Methylendioxy oder Ethylendioxy,
Het einen ein- oder zweikernigen aromatischen Heterocyclus mit 1 bis 3 N-, O- und/oder S-Atomen, der unsubstituiert oder ein-, zwei- oder dreifach durch Hal und/oder A substituiert sein kann,
bedeuten,
sowie ihrer pharmazeutisch verwendbaren Solvate und Salze, einschließlich deren Mischungen in allen Verhältnissen.

25. Verwendung gemäß einem oder mehreren der Ansprüche 1 bis 24 von Verbindungen der Formel I gemäß Anspruch 1
worin
R¹ -OH oder -OA,
R² H, -OH, -OA, Phenoxy, -O-CO-A, -OSO₃H, -OSO₃A, -OSO₂A oder Hal,
R³ H,
R¹ und R² zusammen auch Methylendioxy oder Ethylendioxy,
Het einen ein- oder zweikernigen aromatischen Heterocyclus mit 1 bis 4 N-, O- und/oder S-Atomen, der unsubstituiert oder ein-, zwei- oder dreifach durch Hal und/oder A substituiert sein kann,
bedeuten,
sowie ihrer pharmazeutisch verwendbaren Solvate und Salze, einschließlich deren Mischungen in allen Verhältnissen.

26. Verwendung gemäß einem oder mehreren der Ansprüche 1 bis 25 von Verbindungen der Formel I gemäß Anspruch 1
worin
R¹ -OH oder -OA,
R² H, -OH, -OA, Phenoxy, -O-CO-A, -OSO₃H, -OSO₃A, -OSO₂A oder Hal,
R³ H,
R¹ und R² zusammen auch Methylendioxy oder Ethylendioxy,
Het unsubstituiertes oder ein- oder zweifach durch Hal und/oder A substituiertes Chromen-2-on-yl, Benzothiazolyl, Thienyl, Pyridinyl, Pyrimidinyl, Pyrazinyl, Indolyl, Furyl, Pyrrolyl, Isoxazolyl, Imidazolyl, Thiazolyl, Triazolyl, Tetrazolyl, Chinolyl oder Isochinolyl,
A unverzweigtes oder verzweigtes Alkyl mit 1-6 C-Atomen, wobei 1-5 H-Atome durch F ersetzt sein können,
bedeuten,
sowie ihrer pharmazeutisch verwendbaren Solvate und Salze, einschließlich deren Mischungen in allen Verhältnissen.

27. Verbindungen der Formel I 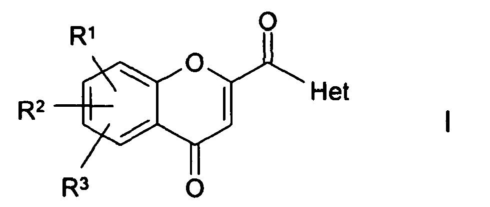 worin
R¹ -OH oder -OA,
R² H, -OH, -OA, Phenoxy, -O-CO-A, -OSO₃H, -OSO₃A, -OSO₂A oder Hal,
R³ H,
R¹ und R² zusammen auch Methylendioxy oder Ethylendioxy,
Het unsubstituiertes oder ein- oder zweifach durch Hal und/oder A substituiertes Chromen-2-on-yl, Benzothiazolyl, Thienyl, Pyridinyl, Pyrimidinyl, Pyrazinyl, Indolyl, Furyl, Pyrrolyl, Isoxazolyl, Imidazolyl, Thiazolyl, Triazolyl, Tetrazolyl, Chinolyl oder Isochinolyl,
A unverzweigtes oder verzweigtes Alkyl mit 1-6 C-Atomen, wobei 1-5 H-Atome durch F ersetzt sein können,
Hal F, Cl, Br oder I
bedeuten,
sowie ihre pharmazeutisch verwendbaren Solvate und Salze, einschließlich deren Mischungen in allen Verhältnissen.

28. Verbindungen der Formel I nach Anspruch 27,
worin
R¹ -OH oder -OA,
R² H, -OH, -OA oder Hal,
R³ H,
R¹ und R² zusammen auch Methylendioxy oder Ethylendioxy,
Het unsubstituiertes oder ein- oder zweifach durch Hal und/oder A substituiertes Benzothiazolyl, Pyridinyl, Pyrimidinyl, Pyrazinyl, Indolyl, Furyl, Pyrrolyl, Isoxazolyl, Imidazolyl oder Thiazolyl,
A unverzweigtes oder verzweigtes Alkyl mit 1-6 C-Atomen, wobei 1-5 H-Atome durch F ersetzt sein können,
bedeuten,
sowie ihre pharmazeutisch verwendbaren Solvate und Salze, einschließlich deren Mischungen in allen Verhältnissen.

29. Verbindungen gemäß Anspruch 27 ausgewählt aus der Gruppe
6-Hydroxy-2-(1-methyl-1*H*-imidazol-2-carbonyl)-chromen-4on,
5,7-Dihydroxy-2-(1-methyl-1*H*-imidazol-2-carbonyl)-chromen-4on,
7-Hydroxy-2-(1-methyl-1*H*-imidazol-2-carbonyl)-chromen-4on,
6-(1-Methyl-1*H*-imidazol-2-carbonyl)-[1,3]dioxolo[4,5*g*]chromen-8-on,
5,7-Dihydroxy-2-(pyridin-2-carbonyl)-chromen-4-on,
6-Hydroxy-2-(pyridin-2-carbonyl)-chromen-4-on,
6-Hydroxy-2-(3,5-dichlorpyrazin-2-carbonyl)-chromen-4-on,
6-Hydroxy-2-(benzothiazolyl-2-carbonyl)-chromen-4-on,
sowie ihre pharmazeutisch verwendbaren Solvate und Salze, einschließlich deren Mischungen in allen Verhältnissen.

30. Arzneimittel, enthaltend mindestens eine Verbindung der Formel I nach einem oder mehreren der Ansprüche 27 bis 29 und/oder ihre pharmazeutisch verwendbaren Solvate und Salze, einschließlich deren Mischungen in allen Verhältnissen, sowie gegebenenfalls Träger- und/oder Hilfsstoffe.

31. Arzneimittel enthaltend mindestens eine Verbindung der Formel I gemäß einem oder mehreren der Ansprüche 27 bis 29 und/oder ihre pharmazeutisch verwendbaren Solvate und Salze, einschließlich deren Mischungen in allen Verhältnissen, und mindestens einen weiteren Arzneimittelwirkstoff.

32. Set (Kit), bestehend aus getrennten Packungen von
(a) einer wirksamen Menge an einer Verbindung der Formel I gemäß einem oder mehreren der Ansprüche 27 bis 29 und/oder ihrer pharmazeutisch verwendbaren Solvate und Salze, einschließlich deren Mischungen in allen Verhältnissen,
und
(b) einer wirksamen Menge eines weiteren Arzneimittelswirkstoffs.

33. Verfahren zur Herstellung von Verbindungen der Formel I nach den Ansprüchen 27-29 sowie ihrer pharmazeutisch verwendbaren Solvate und Salze, **dadurch gekennzeichnet, daß** man
a) zunächst eine Verbindung der Formel II 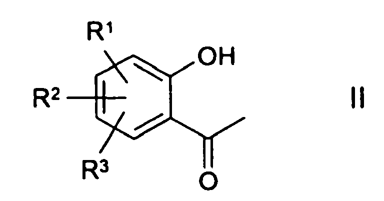 worin
R¹, R², R³ die in Anspruch 27 angegebene Bedeutung haben, mit einer Verbindung der Formel III 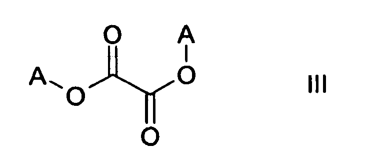 worin A Alkyl mit 1, 2, 3, 4, 5 oder 6 C-Atomen bedeutet, zu einer Verbindung der Formel IV 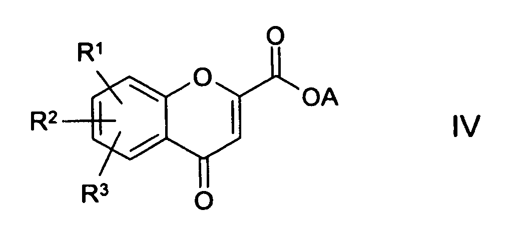 worin R¹, R², R³ die in Anspruch 27 angegebene Bedeutung haben, und A Alkyl mit 1, 2, 3, 4, 5 oder 6 C-Atomen bedeutet,
umsetzt,
b) anschließend den Ester IV mit einer Verbindung der Formel V
M-Het V
worin Het die in Anspruch 27 angegebene Bedeutung hat,
und M Natrium, Kalium oder Lithium bedeutet
zu einer Verbindung der Formel I umsetzt
und/oder
c) eine Base oder Säure der Formel I in eines ihrer Salze umwandelt.

34. Verwendung von Verbindungen der Formel I gemäß einem oder mehreren der Ansprüche 27-29 und/oder ihrer physiologisch unbedenklichen Salze und Solvate zur Herstellung eines Arzneimittels zur Behandlung von Krankheiten und/oder Fehlfunktionen, die durch oxidative Stressbedingungen charakterisiert sind.

35. Verwendung nach Anspruch 34, wobei es sich bei den Krankheiten und/oder Fehlfunktionen um Gedächtnisverlust und neurodegenerative Erkrankungen handelt.

36. Verwendung von Verbindungen gemäß einem oder mehreren der Ansprüche 27-29 und/oder ihre physiologisch unbedenklichen Salze und Solvate als Nahrungsmittelzusatzstoffe.

37. Verwendung von Verbindungen gemäß einem oder mehreren der Ansprüche 27-29 und/oder ihre physiologisch unbedenklichen Salze und Solvate in kosmetischen Formulierungen.

38. Verwendung nach Anspruch 37 zum Schutz der Streßproteine der Haut.

39. Verwendung nach Anspruch 37 oder 38 in Form einer topischen Zusammensetzung.

40. Verwendung nach Anspruch 37, 38 oder 39, **dadurch gekennzeichnet, daß** mindestens eine verwendete Verbindung in einer topischen Zusammensetzung in einer Menge von 0,0001 bis 50 Gew.-%, bezogen auf die Zusammensetzung, vorliegt.

## Claims

1. Use of compounds of the formula I 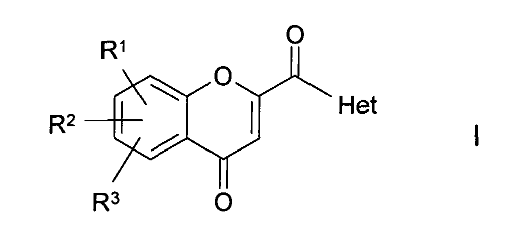 in which
R¹ denotes H, -OH, -OA, phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO or SO₂NH₂,
R² denotes H, -OH, -OA, phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO or SO₂NH₂,
R³ denotes H, -OH, -OA, phenoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO or SO₂NH₂,
R¹ and R² together also denote methylenedioxy or ethylenedioxy,
Het denotes a mono- or bicyclic saturated, unsaturated or aromatic heterocyclic ring having 1 to 4 N, O and/or S atoms, which may be unsubstituted or mono-, di- or trisubstituted by carbonyl oxygen, =S, =NH, Hal, A, -(CH₂)ₒ-Ar, -(CH₂)ₒ-cycloalkyl, -(CH₂)ₒ-OH, -(CH₂)ₒ-NH₂, NO₂, CN, -(CH₂)ₒ-COOH, -(CH₂)ₒ-COOA, -(CH₂)ₒ-CONH₂, -(CH₂)ₒ-NHCOA, NHCONH₂, -(CH₂)ₒ-NHSO₂A, CHO, COA', SO₂NH₂ and/or S(O)ₒA,
Ar denotes phenyl, naphthyl or biphenyl, each of which is unsubstituted or mono-, di- or trisubstituted by Hal, A, OH, OA, NH₂, NO₂, CN, COOH, COOA, CONH₂, NHCOA, NHCONH₂, NHSO₂A, CHO, COA, SO₂NH₂ or S(O)ₒA,
A denotes unbranched or branched alkyl having 1-10 C atoms, where 1-7 H atoms may be replaced by F,
A' denotes alkyl having 1 to 6 C atoms or benzyl,
Hal denotes F, Cl, Br or I,
o denotes 0, 1 or 2,
and pharmaceutically usable solvates and salts thereof, including mixtures thereof in all ratios,
for the preparation of a medicament for the treatment of diseases in which the inhibition, regulation and/or modulation of kinase signal transduction plays a role.

2. Use according to Claim 1, where the kinases are selected from the group of the tyrosine kinases and/or Raf kinases.

3. Use according to Claim 1 or 2 of compounds of the formula I according to Claim 1, and pharmaceutically usable solvates and salts thereof, including mixtures thereof in all ratios,
for the preparation of a medicament for the treatment of diseases which are influenced by inhibition of tyrosine kinases by the compounds of the formula I.

4. Use according to Claim 3, where the disease to be treated is a solid tumour.

5. Use according to Claim 4, where the solid tumour originates from the group brain tumour, tumour of the urogenital tract, tumour of the lymphatic system, stomach tumour, laryngeal tumour and lung tumour.

6. Use according to Claim 5, where the solid tumour originates from the group monocytic leukaemia, lung adenocarcinoma, small-cell lung carcinomas, pancreatic cancer, glioblastomas and breast carcinoma.

7. Use according to Claim 3 for the treatment of a disease in which angiogenesis is implicated.

8. Use according to Claim 7, where the disease is an ocular disease.

9. Use according to Claim 3 for the treatment of retinal vascularisation, diabetic retinopathy, age-induced macular degeneration and/or inflammatory diseases.

10. Use according to Claim 9, where the inflammatory disease originates from the group rheumatoid arthritis, psoriasis, contact dermatitis and delayed hypersensitivity reaction.

11. Use according to Claim 3 for the treatment of bone pathologies, where the bone pathology originates from the group osteosarcoma, osteoarthritis and rickets.

12. Use according to Claim 3 of compounds of the formula I and/or physiologically acceptable salts and solvates thereof for the preparation of a medicament for the treatment of solid tumours, where a therapeutically effective amount of a compound according to Claim 1 is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

13. Use according to Claim 3 of compounds of the formula I and/or physiologically acceptable salts and solvates thereof for the preparation of a medicament for the treatment of solid tumours, where a therapeutically effective amount of a compound according to Claim 1 is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

14. Use according to Claim 1 or 2 of compounds of the formula I according to Claim 1, and pharmaceutically usable solvates and salts thereof, including mixtures thereof in all ratios,
for the preparation of a medicament for the treatment of diseases which are caused, mediated and/or propagated by Raf kinases.

15. Use according to Claim 14, where the Raf kinase is selected from the group consisting of A-Raf, B-Raf and Raf-1.

16. Use according to Claim 14, where the diseases are selected from the group of the hyperproliferative and non-hyperproliferative diseases.

17. Use according to Claim 14 or 16, where the disease is cancer.

18. Use according to Claim 14 or 16, where the disease is non-cancerous.

19. Use according to Claim 14, 16 or 18, where the non-cancerous diseases are selected from the group consisting of psoriasis, arthritis, inflammation, endometriosis, scarring, benign prostatic hyperplasia, immunological diseases, autoimmune diseases and immunodeficiency diseases.

20. Use according to one of Claims 14, 16 or 17, where the diseases are selected from the group consisting of brain cancer, lung cancer, squamous cell cancer, bladder cancer, gastric cancer, pancreatic cancer, hepatic cancer, renal cancer, colorectal cancer, breast cancer, head cancer, neck cancer, oesophageal cancer, gynaecological cancer, thyroid cancer, lymphoma, chronic leukaemia and acute leukaemia.

21. Use according to one or more of Claims 1 to 20 of compounds of the formula I according to Claim 1
in which
R¹ denotes H, -OH, -OA, phenoxy, -O-CO-A, -OSO₃H, -OSO₃A, -OSO₂A or Hal,
and pharmaceutically usable solvates and salts thereof, including mixtures thereof in all ratios.

22. Use according to one or more of Claims 1 to 21 of compounds of the formula I according to Claim 1
in which
R¹ denotes H, -OH, -OA, phenoxy, -O-CO-A, -OSO₃H, -OSO₃A, -OSO₂A or Hal,
R² denotes H, -OH, -OA, phenoxy, -O-CO-A, -OSO₃H, -OSO₃A, -OSO₂A or Hal,
R³ denotes H,
R¹ and R² together also denote methylenedioxy or ethylenedioxy,
and pharmaceutically usable solvates and salts thereof, including mixtures thereof in all ratios.

23. Use according to one or more of Claims 1 to 22 of compounds of the formula I according to Claim 1
in which
R¹ denotes -OH, -OA, phenoxy, -O-CO-A, -OSO₃H, -OSO₃A, -OSO₂A or Hal,
R² denotes H, -OH, -OA, phenoxy, -O-CO-A, -OSO₃H, -OSO₃A, -OSO₂A or Hal,
R³ denotes H,
R¹ and R² together also denote methylenedioxy or ethylenedioxy,
Het denotes a mono- or bicyclic saturated, unsaturated or aromatic heterocyclic ring having 1 to 4 N, O and/or S atoms, which may be unsubstituted or mono-, di- or trisubstituted by Hal and/or A,
and pharmaceutically usable solvates and salts thereof, including mixtures thereof in all ratios.

24. Use according to one or more of Claims 1 to 23 of compounds of the formula I according to Claim 1
in which
R¹ denotes -OH, -OA, phenoxy, -O-CO-A, -OSO₃H, -OSO₃A, -OSO₂A or Hal,
R² denotes H, -OH, -OA, phenoxy, -O-CO-A, -OSO₃H, -OSO₃A, -OSO₂A or Hal,
R³ denotes H,
R¹ and R² together also denote methylenedioxy or ethylenedioxy,
Het denotes a mono- or bicyclic aromatic heterocyclic ring having 1 to 3 N, O and/or S atoms, which may be un-substituted or mono-, di- or trisubstituted by Hal and/or A,
and pharmaceutically usable solvates and salts thereof, including mixtures thereof in all ratios.

25. Use according to one or more of Claims 1 to 24 of compounds of the formula I according to Claim 1
in which
R¹ denotes -OH or -OA,
R² denotes H, -OH, -OA, phenoxy, -O-CO-A, -OSO₃H, -OSO₃A, -OSO₂A or Hal,
R³ denotes H,
R¹ and R² together also denote methylenedioxy or ethylenedioxy,
Het denotes a mono- or bicyclic aromatic heterocyclic ring having 1 to 4 N, O and/or S atoms, which may be un-substituted or mono-, di- or trisubstituted by Hal and/or A,
and pharmaceutically usable solvates and salts thereof, including mixtures thereof in all ratios.

26. Use according to one or more of Claims 1 to 25 of compounds of the formula I according to Claim 1
in which
R¹ denotes -OH or -OA,
R² denotes H, -OH, -OA, phenoxy, -O-CO-A, -OSO₃H, -OSO₃A, -OSO₂A or Hal,
R³ denotes H,
R¹ and R² together also denote methylenedioxy or ethylenedioxy,
Het denotes chromen-2-onyl, benzothiazolyl, thienyl, pyridinyl, pyrimidinyl, pyrazinyl, indolyl, furyl, pyrrolyl, isoxazolyl, imidazolyl, thiazolyl, triazolyl, tetrazolyl, quinolyl or isoquinolyl, each of which is unsubstituted or mono- or di-substituted by Hal and/or A,
A denotes unbranched or branched alkyl having 1-6 C atoms, where 1-5 H atoms may be replaced by F,
and pharmaceutically usable solvates and salts thereof, including mixtures thereof in all ratios.

27. Compounds of the formula I 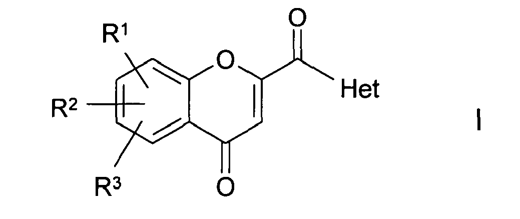 in which
R¹ denotes -OH or -OA,
R² denotes H, -OH, -OA, phenoxy, -O-CO-A, -OSO₃H, -OSO₃A, -OSO₂A or Hal,
R³ denotes H,
R¹ and R² together also denote methylenedioxy or ethylenedioxy,
Het denotes chromen-2-onyl, benzothiazolyl, thienyl, pyridinyl, pyrimidinyl, pyrazinyl, indolyl, furyl, pyrrolyl, isoxazolyl, imidazolyl, thiazolyl, triazolyl, tetrazolyl, quinolyl or isoquinolyl, each of which is unsubstituted or mono- or di-substituted by Hal and/or A,
A denotes unbranched or branched alkyl having 1-6 C atoms, where 1-5 H atoms may be replaced by F,
Hal denotes F, Cl, Br or I,
and pharmaceutically usable solvates and salts thereof, including mixtures thereof in all ratios.

28. Compounds of the formula I according to Claim 27
in which
R¹ denotes -OH or -OA,
R² denotes H, -OH, -OA or Hal,
R³ denotes H,
R¹ and R² together also denote methylenedioxy or ethylenedioxy,
Het denotes benzothiazolyl, pyridinyl, pyrimidinyl, pyrazinyl, indolyl, furyl, pyrrolyl, isoxazolyl, imidazolyl or thiazolyl, each of which is unsubstituted or mono- or disubstituted by Hal and/or A,
A denotes unbranched or branched alkyl having 1-6 C atoms, where 1-5 H atoms may be replaced by F,
and pharmaceutically usable solvates and salts thereof, including mixtures thereof in all ratios.

29. Compounds according to Claim 27 selected from the group
6-hydroxy-2-(1-methyl-1H-imidazole-2-carbonyl)chromen-4-one,
5,7-dihydroxy-2-(1-methyl-1*H*-imidazole-2-carbonyl)chromen-4-one,
7-hydroxy-2-(1 -methyl-1*H*-imidazole-2-carbonyl)chromen-4-one,
6-(1-methyl-1*H*-imidazole-2-carbonyl)-1,3-dioxolo[4,5*g*]chromen-8-one,
5,7-dihydroxy-2-(pyridine-2-carbonyl)chromen-4-one,
6-hydroxy-2-(pyridine-2-carbonyl)chromen-4-one,
6-hydroxy-2-(3,5-dichloropyrazine-2-carbonyl)chromen-4-one,
6-hydroxy-2-(benzothiazolyl-2-carbonyl)chromen-4-one,
and pharmaceutically usable solvates and salts thereof, including mixtures thereof in all ratios.

30. Medicaments comprising at least one compound of the formula I according to one or more of Claims 27 to 29 and/or pharmaceutically usable solvates and salts thereof, including mixtures thereof in all ratios, and optionally excipients and/or adjuvants.

31. Medicaments comprising at least one compound of the formula I according to one or more of Claims 27 to 29 and/or pharmaceutically usable solvates and salts thereof, including mixtures thereof in all ratios, and at least one further medicament active ingredient.

32. Set (kit) consisting of separate packs of
(a) an effective amount of a compound of the formula I according to one or more of Claims 27 to 29 and/or pharmaceutically usable solvates and salts thereof, including mixtures thereof in all ratios,
and
(b) an effective amount of a further medicament active ingredient.

33. Process for the preparation of compounds of the formula I according to Claims 27-29 and pharmaceutically usable solvates and salts thereof, **characterised in that**
a) firstly a compound of the formula II 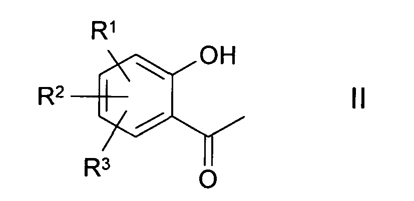 in which
R¹, R² and R³ have the meaning indicated in Claim 27, is reacted with a compound of the formula III 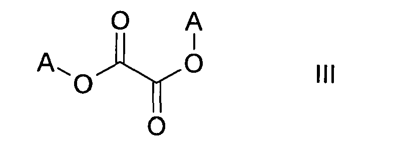 in which A denotes alkyl having 1, 2, 3, 4, 5 or 6 C atoms, to give a compound of the formula IV 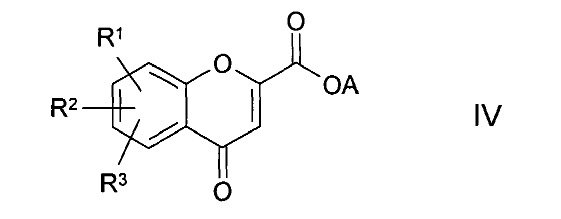 in which R¹, R², R³ have the meaning indicated in Claim 27, and A denotes alkyl having 1, 2, 3, 4, 5 or 6 C atoms,
b) subsequently the ester IV is reacted with a compound of the formula V
M-Het V
in which Het has the meaning indicated in Claim 27,
and M denotes sodium, potassium or lithium,
to give a compound of the formula I
and/or
c) a base or acid of the formula I is converted into one of its salts.

34. Use of compounds of the formula I according to one or more of Claims 27-29 and/or physiologically acceptable salts and solvates thereof for the preparation of a medicament for the treatment of diseases and/or disorders which are **characterised by** oxidative stress conditions.

35. Use according to Claim 34, where the diseases and/or disorders are memory loss and neurodegenerative diseases.

36. Use of compounds according to one or more of Claims 27-29 and/or physiologically acceptable salts and solvates thereof as food additives.

37. Use of compounds according to one or more of Claims 27-29 and/or physiologically acceptable salts and solvates thereof in cosmetic formulations.

38. Use according to Claim 37 for protection of the stress proteins of the skin.

39. Use according to Claim 37 or 38 in the form of a topical composition.

40. Use according to Claim 37, 38 or 39, **characterised in that** at least one compound used is present in a topical composition in an amount of 0.0001 to 50% by weight, based on the composition.

## Revendications

1. Utilisation de composés de la formule I 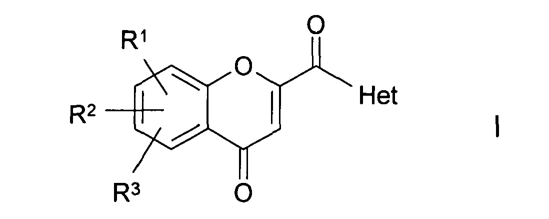 dans laquelle
R¹ représente H, -OH, -OA, phénoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO ou SO₂NH₂,
R² représente H, -OH, -OA, phénoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO ou SO₂NH₂,
R³ représente H, -OH, -OA, phénoxy, Ar, -O-CO-A, SO₃H, SO₃A, -OSO₃H, -OSO₃A, -OSO₂A, SO₂A, Hal, COOH, COOA, CONH₂, NHSO₂A, COA, CHO ou SO₂NH₂,
R¹ et R² ensemble, représentent également méthylènedioxy ou éthylènedioxy,
Het représente un cycle hétérocyclique saturé, insaturé ou aromatique mono- ou bicyclique comportant de 1 à 4 atomes de N, O et/ ou S, qui peut être non substitué ou mono-, di- ou trisubstitué par oxygène carbonyle, =S, =NH, Hal, A, -(CH₂)ₒ-Ar, -(CH₂)ₒ-cycloalkyle, -(CH₂)ₒ-OH, -(CH₂)ₒ-NH₂, NO₂, CN, -(CH₂)ₒ-COOH, -(CH₂)ₒ-COOA, -(CH₂)ₒ-CONH₂, -(CH₂)ₒ-NHCOA, NHCONH₂, -(CH₂)ₒ-NHSO₂A, CHO, COA', SO₂NH₂ et/ou S(O)ₒA,
Ar représente phényle, naphtyle ou biphényle dont chacun est non substitué ou mono-, di- ou trisubstitué par Hal, A, OH, OA, NH₂, NO₂, CN, COOH, COOA, CONH₂, NHCOA, NHCONH₂, NHSO₂A, CHO, COA, SO₂NH₂ ou S(O)ₒA,
A représente alkyle non ramifié ou ramifié comportant de 1 à 10 atomes de C, où 1 à 7 atomes de H peuvent être remplacés par F,
A' représente alkyle comportant de 1 à 6 atomes de C ou benzyle,
Hal représente F, Cl, Br ou I,
o représente 0, 1 ou 2,
et de leurs solvates et sels utilisables pharmaceutiquement, y compris des mélanges afférents selon tous rapports,
pour la préparation d'un médicament pour le traitement de maladies au niveau desquelles l'inhibition, la régulation et/ou la modulation de transduction de signal kinase joue(nt) un rôle.

2. Utilisation selon la revendication 1, dans laquelle les kinases sont choisies parmi le groupe des kinases tyrosine et/ou des kinases Raf.

3. Utilisation selon la revendication 1 ou 2 de composés de la formule I selon la revendication 1 et de leurs solvates et sels utilisables pharmaceutiquement, y compris des mélanges afférents selon tous rapports,
pour la préparation d'un médicament pour le traitement de maladies qui sont influencées par l'inhibition des kinases tyrosine par les composés de la formule I.

4. Utilisation selon la revendication 3, dans laquelle la maladie à traiter est une tumeur solide.

5. Utilisation selon la revendication 4, dans laquelle la tumeur solide est issue du groupe comprenant la tumeur du cerveau, la tumeur du tractus urogénital, la tumeur du système lymphatique, la tumeur de l'estomac, la tumeur du larynx et la tumeur des poumons.

6. Utilisation selon la revendication 5, dans laquelle la tumeur solide est issue du groupe comprenant la leucémie monocytique, l'adénocarcinome des poumons, les carcinomes du poumon à petites cellules, le cancer du pancréas, les glioblastomes et le carcinome du sein.

7. Utilisation selon la revendication 3 pour le traitement d'une maladie au niveau de laquelle une angiogénèse est impliquée.

8. Utilisation selon la revendication 7, dans laquelle la maladie est une maladie oculaire.

9. Utilisation selon la revendication 3 pour le traitement de la vascularisation rétinienne, de la rétinopathie diabétique, de la dégénérescence maculaire liée à l'âge et/ou de maladies inflammatoires.

10. Utilisation selon la revendication 9, dans laquelle la maladie inflammatoire est issue du groupe comprenant l'arthrite rhumatoïde, le psoriasis, l'eczéma de contact et la réaction d'hypersensibilité retardée.

11. Utilisation selon la revendication 3 pour le traitement de pathologies des os où la pathologie des os est issue du groupe comprenant l'ostéosarcome, l'ostéoarthrite et le rachitisme.

12. Utilisation selon la revendication 3 de composés de la formule I et/ou de leurs sels et solvates acceptables physiologiquement pour la préparation d'un médicament pour le traitement de tumeurs solides, où une quantité efficace thérapeutiquement d'un composé selon la revendication 1 est administrée en combinaison avec un composé issu du groupe comprenant 1) un modulateur de récepteur d'oestrogène, 2) un modulateur de récepteur d'androgène, 3) un modulateur de récepteur de rétinoïde, 4) un agent cytotoxique, 5) un agent antiprolifération, 6) un inhibiteur de transférase de protéine prényle, 7) un inhibiteur de réductase HMG-CoA, 8) un inhibiteur de protéase HIV, 9) un inhibiteur de transcriptase inverse et 10) d'autres inhibiteurs d'angiogénèse.

13. Utilisation selon la revendication 3 de composés de la formule I et/ou de leurs sels et solvates acceptables physiologiquement pour la préparation d'un médicament pour le traitement de tumeurs solides, où une quantité efficace thérapeutiquement d'un composé selon la revendication 1 est administrée en combinaison avec une radiothérapie et un composé issu du groupe comprenant 1) un modulateur de récepteur d'oestrogène, 2) un modulateur de récepteur d'androgène, 3) un modulateur de récepteur de rétinoïde, 4) un agent cytotoxique, 5) un agent antiprolifération, 6) un inhibiteur de transférase de protéine prényle, 7) un inhibiteur de réductase HMG-CoA, 8) un inhibiteur de protéase HIV, 9) un inhibiteur de transcriptase inverse et 10) d'autres inhibiteurs d'angiogénèse.

14. Utilisation selon la revendication 1 ou 2 de composés de la formule I selon la revendication 1 et de leurs sels et solvates utilisables pharmaceutiquement, y compris des mélanges afférents selon tous rapports,
pour la préparation d'un médicament pour le traitement de maladies qui sont générées, favorisées et/ou propagées par des kinases Raf.

15. Utilisation selon la revendication 14, dans laquelle la kinase Raf est choisie parmi le groupe comprenant A-Raf, B-Raf et Raf-1.

16. Utilisation selon la revendication 14, dans laquelle les maladies sont choisies parmi le groupe des maladies hyper-prolifératives et non hyper-prolifératives.

17. Utilisation selon la revendication 14 ou 16, dans laquelle la maladie est le cancer.

18. Utilisation selon la revendication 14 ou 16, dans laquelle la maladie est non cancéreuse.

19. Utilisation selon la revendication 14, 16 ou 18, dans laquelle les maladies non cancéreuses sont choisies parmi le groupe comprenant le psoriasis, l'arthrite, l'inflammation, l'endométriose, la cicatrisation, l'hyperplasie prostatique bénigne, les maladies immunologiques, les maladies auto-immunes et les maladies immunodéficitaires.

20. Utilisation selon l'une des revendications 14, 16 ou 17, dans laquelle les maladies sont choisies parmi le groupe comprenant le cancer du cerveau, le cancer des poumons, le cancer des cellules squameuses, le cancer de la vessie, le cancer de l'estomac, le cancer du pancréas, le cancer du foie, le cancer du rein, le cancer du colon rectum, le cancer du sein, le cancer de la tête, le cancer du cou, le cancer de l'oesophage, le cancer des organes gynécologiques, le cancer de la thyroïde, le lymphome, la leucémie chronique et la leucémie aiguë.

21. Utilisation selon une ou plusieurs des revendications 1 à 20 de composés de la formule I selon la revendication 1
dans laquelle
R¹ représente H, -OH, -OA, phénoxy, -O-CO-A, -OSO₃H, -OSO₃A, -OSO₂A ou Hal,
et de leurs solvates et sels utilisables pharmaceutiquement, y compris des mélanges afférents selon tous rapports.

22. Utilisation selon une ou plusieurs des revendications 1 à 21 de composés de la formule I selon la revendication 1
dans laquelle
R¹ représente H, -OH, -OA, phénoxy, -O-CO-A, -OSO₃H, -OSO₃A, -OSO₂A ou Hal,
R² représente H, -OH, -OA, phénoxy, -O-CO-A, -OSO₃H, -OSO₃A, -OSO₂A ou Hal,
R³ représente H,
R¹ et R² ensemble, représentent également méthylènedioxy ou éthylènedioxy
et de leurs solvates et sels utilisables pharmaceutiquement, y compris des mélanges afférents selon tous rapports.

23. Utilisation selon une ou plusieurs des revendications 1 à 22 de composés de la formule I selon la revendication 1
dans laquelle
R¹ représente -OH, -OA, phénoxy, -O-CO-A, -OSO₃H, -OSO₃A, -OSO₂A ou Hal,
R² représente H, -OH, -OA, phénoxy, -O-CO-A, -OSO₃H, -OSO₃A, -OSO₂A ou Hal,
R³ représente H,
R¹ et R² ensemble, représentent également méthylènedioxy ou éthylènedioxy,
Het représente un cycle hétérocyclique saturé, insaturé ou aromatique mono- ou bicyclique comportant de 1 à 4 atomes de N, O et/ou S, qui peut être non substitué ou mono-, di- ou trisubstitué par Hal et/ou A,
et de leurs solvates et sels utilisables pharmaceutiquement, y compris des mélanges afférents selon tous rapports.

24. Utilisation selon une ou plusieurs des revendications 1 à 23 de composés de la formule I selon la revendication 1
dans laquelle
R¹ représente -OH, -OA, phénoxy, -O-CO-A, -OSO₃H, -OSO₃A, -OSO₂A ou Hal,
R² représente H, -OH, -OA, phénoxy, -O-CO-A, -OSO₃H, -OSO₃A, -OSO₂A ou Hal,
R³ représente H,
R¹ et R² ensemble, représentent également méthylènedioxy ou éthylènedioxy,
Het représente un cycle hétérocyclique aromatique mono- ou bicyclique comportant de 1 à 3 atomes de N, O et/ou S, qui peut être non substitué ou mono-, di- ou trisubstitué par Hal et/ou A,
et de leurs solvates et sels utilisables pharmaceutiquement, y compris des mélanges afférents selon tous rapports.

25. Utilisation selon une ou plusieurs des revendications 1 à 24 de composés de la formule I selon la revendication 1
dans laquelle
R¹ représente -OH ou -OA,
R² représente H, -OH, -OA, phénoxy, -O-CO-A, -OSO₃H, -OSO₃A, -OSO₂A ou Hal,
R³ représente H,
R¹ et R² ensemble, représentent également méthylènedioxy ou éthylènedioxy,
Het représente un cycle hétérocyclique aromatique mono- ou bicyclique comportant de 1 à 4 atomes de N, O et/ou S, qui peut être non substitué ou mono-, di- ou trisubstitué par Hal et/ou A,
et de leurs solvates et sels utilisables pharmaceutiquement, y compris des mélanges afférents selon tous rapports.

26. Utilisation selon une ou plusieurs des revendications 1 à 25 de composés de la formule I selon la revendication 1
dans laquelle
R¹ représente -OH ou -OA,
R² représente H, -OH, -OA, phénoxy, -O-CO-A, -OSO₃H, -OSO₃A, -OSO₂A ou Hal,
R³ représente H,
R¹ et R² ensemble, représentent également méthylènedioxy ou éthylènedioxy,
Het représente chromen-2-onyle, benzothiazolyle, thiényle, pyridinyle, pyrimidinyle, pyrazinyle, indolyle, furyle, pyrrolyle, isoxazolyle, imidazolyle, thiazolyle, triazolyle, tétrazolyle, quinolyle ou isoquinolyle dont chacun est non substitué ou mono- ou disubstitué par Hal et/ou A,
A représente alkyle non ramifié ou ramifié comportant de 1 à 6 atomes de C, où 1 à 5 atomes de H peuvent être remplacés par F,
et de leurs solvates et sels utilisables pharmaceutiquement, y compris des mélanges afférents selon tous rapports.

27. Composés de la formule I 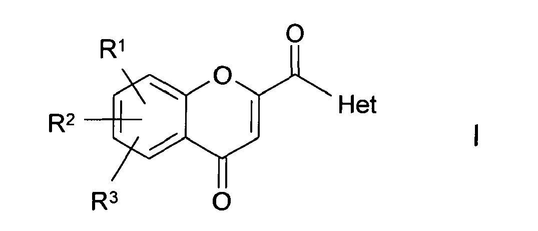 dans laquelle
R¹ représente -OH ou -OA,
R² représente H, -OH, -OA, phénoxy, -O-CO-A, -OSO₃H, -OSO₃A, -OSO₂A ou Hal,
R³ représente H,
R¹ et R² ensemble, représentent également méthylènedioxy ou éthylènedioxy,
Het représente chromen-2-onyle, benzothiazolyle, thiényle, pyridinyle, pyrimidinyle, pyrazinyle, indolyle, furyle, pyrrolyle, isoxazolyle, imidazolyle, thiazolyle, triazolyle, tétrazolyle, quinolyle ou isoquinolyle dont chacun est non substitué ou mono- ou disubstitué par Hal et/ou A,
A représente alkyle non ramifié ou ramifié comportant de 1 à 6 atomes de C, où 1 à 5 atomes de H peuvent être remplacés par F,
Hal représente F, Cl, Br ou I,
et leurs solvates et sels utilisables pharmaceutiquement, y compris des mélanges afférents selon tous rapports.

28. Composés de la formule I selon la revendication 27
dans laquelle
R¹ représente -OH ou -OA,
R² représente H, -OH, -OA ou Hal,
R³ représente H,
R¹ et R² ensemble, représentent également méthylènedioxy ou éthylènedioxy,
Het représente benzothiazolyle, pyridinyle, pyrimidinyle, pyrazinyle, indolyle, furyle, pyrrolyle, isoxazolyle, imidazolyle ou thiazolyle dont chacun est non substitué ou mono- ou di-substitué par Hal et/ou A,
A représente alkyle non ramifié ou ramifié comportant de 1 à 6 atomes de C, où 1 à 5 atomes de H peuvent être remplacés par F,
et leurs solvates et sels utilisables pharmaceutiquement, y compris des mélanges afférents selon tous rapports.

29. Composés selon la revendication 27 choisis parmi le groupe
6-hydroxy-2-(1-méthyl-1*H*-imidazole-2-carbonyl)chromen-4-one,
5,7-dihydroxy-2-(1-méthyt-1H-imidazole-2-carbonyl)chromen-4-one,
7-hydroxy-2-(1-méthyl-1*H*-imidazole-2-carbonyl)chromen-4-one,
6-(1-méthyl-1*H*-imidazole-2-carbonyl)-1,3-dioxolo[4,5*g*]chromen-8-one,
5,7-dihydroxy-2-(pyridine-2-carbonyl)chromen-4-one,
6-hydroxy-2-(pyridine-2-carbonyl)chromen-4-one,
6-hydroxy-2-(3,5-dichloropyrazine-2-carbonyl)chromen-4-one,
6-hydroxy-2-(benzothiazolyl-2-carbonyl)chromen-4-one,
et leurs solvates et sels utilisables pharmaceutiquement, y compris des mélanges afférents selon tous rapports.

30. Médicaments comprenant au moins un composé de la formule I selon une ou plusieurs des revendications 27 à 29 et/ou leurs solvates et sels utilisables pharmaceutiquement, y compris des mélanges afférents selon tous rapports et en option, des excipients et/ou des adjuvants.

31. Médicaments comprenant au moins un composé de la formule I selon une ou plusieurs des revendications 27 à 29 et/ou leurs solvates et sels utilisables pharmaceutiquement, y compris des mélanges afférents selon tous rapports et au moins un autre ingrédient actif de médicament.

32. Ensemble (kit) constitué par des groupements séparés de
a) une quantité efficace d'un composé de la formule I selon une ou plusieurs des revendications 27 à 29 et/ou de leurs solvates et sels utilisables pharmaceutiquement, y compris des mélanges afférents selon tous rapports et
b) une quantité efficace d'un autre ingrédient actif de médicament.

33. Procédé pour la préparation de composés de la formule I selon les revendications 27-29 et de leurs solvates et sels utilisables pharmaceutiquement, **caractérisé en ce que**
a) en premier lieu, un composé de la formule II 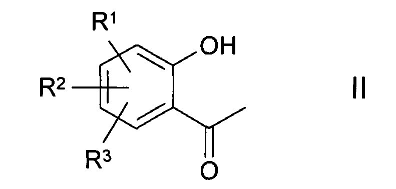 dans laquelle
R¹, R² et R³ présentent la signification indiquée dans la revendication 27
est amené à réagir avec un composé de la formule III 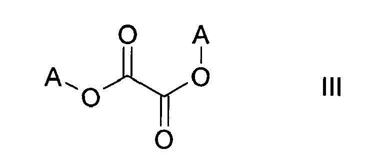 dans laquelle A représente alkyle comportant 1, 2, 3, 4, 5 ou 6 atomes de C,
afin d'obtenir un composé de la formule IV 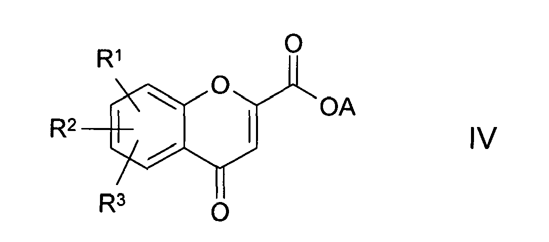 dans laquelle R¹, R², R³ présentent la signification indiquée dans la revendication 27,
et A représente alkyle comportant 1, 2, 3, 4, 5 ou 6 atomes de C,
b) ensuite, l'ester IV est amené à réagir avec un composé de la formule V
M-Het V
dans laquelle Het présente la signification indiquée dans la revendication 27
et M représente sodium, potassium ou lithium,
pour obtenir un composé de la formule I
et/ou
c) une base ou un acide de la formule I est converti(e) selon l'un de ses sels.

34. Utilisation de composés de la formule I selon une ou plusieurs des revendications 27-29 et/ou de leurs sels et solvates acceptables physiologiquement pour la préparation d'un médicament pour le traitement de maladies et/ou de troubles qui sont **caractérisés par** des conditions de stress oxydatif.

35. Utilisation selon la revendication 34, dans laquelle les maladies et/ou les troubles sont des maladies de perte de mémoire et neurodégénératives.

36. Utilisation de composés selon une ou plusieurs des revendications 27-29 et/ou de leurs sels et solvates acceptables physiologiquement en tant qu'additifs alimentaires.

37. Utilisation de composés selon une ou plusieurs des revendications 27-29 et/ou de leurs sels et solvates acceptables physiologiquement dans des formulations cosmétiques.

38. Utilisation selon la revendication 37, pour la protection des protéines de choc thermique de la peau.

39. Utilisation selon la revendication 37 ou 38 sous la forme d'une composition topique.

40. Utilisation selon la revendication 37, 38 ou 39, **caractérisée en ce qu'**au moins un composé utilisé est présent dans une composition topique selon une quantité de 0,0001 à 50% en poids, sur la base de la composition.
